# EUROPEAN PATENT APPLICATION

(11) **EP 4 414 456 A1**
(43) Date of publication of application: **14.08.2024**
(21) Application number: 23305190.3
(22) Date of filing: 13.02.2023
(51) Int. Cl.: C12Q 1/12, C12Q 1/689

(54) **METHOD USING A TRANSCRIPTOMIC SCORE FOR ASSESSING THE CLINICAL OUTCOME OF A PATIENT IN A CLINICAL SETTING, AND MEANS FOR ITS IMPLEMENTATION**

(71) Applicant: BIOMÉRIEUX, 69280 Marcy L'Etoile (FR); Hospices Civils de Lyon, 69002 Lyon (FR); UNIVERSITE CLAUDE BERNARD - LYON 1, 69100 Villeurbanne (FR)
(72) Inventor: LLITJOS, Jean-François, 69160 TASSIN LA DEMI-LUNE (FR); MONNERET, Guillaume, 69008 LYON (FR)
(74) Representative: Plasseraud IP

(57) **Abstract**

The invention relates to a method of determining, from an assayed sample or a population of assayed samples previously drawn from patients, a transcriptomic score (TScore) suitable for assessing a risk of adverse clinical outcome for patients in a clinical setting, said method involving, amongst other steps, the measurement of gene expression values on a patient sample or a population of samples previously obtained from patients for at least two distinct genes selected among a predetermined set of genes, and the determination of a transcriptomic score where the genes are assigned or not assigned a point. The method can be computer-implemented. The genes of the predetermined set of genes can be at least two genes selected among: *ADGRE3, ARL14EP, BPGM, C3AR1, CCNB1IP1, CD177, CD274, CD3D,* CD74, *CIITA, CTLA4, CX3CR1, GNLY, IFNgamma, IL10, IL1R2, IL1RN, IL7R, IP10*/*CXCL10, MDC1, OAS2, S100A9, TAP2, TDRD9,* TNF and *ZAP70.* The method can be implemented on assayed biological sample(s) drawn from patient(s) which have been admitted in a reanimation unit, an intensive or continuing care unit. The invention also relates to a method to classify a sample previously drawn from a patient, in a group reflecting a risk of adverse clinical outcome in a clinical setting, or to identify a patient at risk of adverse clinical outcome in a clinical setting, as well as an *in vitro* or ex *vivo* method of screening whether a drug has the capacity to alleviate an adverse clinical outcome in a patient. The invention also relates to computer means for implementing the invention, and the use of a kit for carrying out a method of the invention.

## Description

### FIELD OF THE INVENTION

The invention relates to the field of investigation methods for determining the potential outcome of the clinical situation of a patient which has been admitted in a care facility, in particular an intensive-care unit (ICU). The invention provides for a method of determining a transcriptomic score (TScore) suitable for assessing a risk of adverse clinical outcome for patients in a clinical setting, said method being based on statistical treatment of measured gene expression values. The method can be computer-implemented. The invention also relates to a method to classify a sample previously drawn from a patient, in particular previously drawn from a patient admitted in an Intensive Care Unit, in a group reflecting a risk of adverse clinical outcome in a clinical setting, and to a method to identify a patient at risk of adverse clinical outcome in a clinical setting. The invention is in particular concerned by the outcome of intensive care unit acquired infection (ICU-AI), notably a nosocomial infection. The invention also relates to an *in vitro* or ex *vivo* method of screening whether a drug has the capacity to alleviate an adverse clinical outcome, based on a stratification of patients with respect to their immune background. The invention also relates to computer means to implement a method carried out by a computer and the use of a kit comprising them.

### BACKGROUND OF THE INVENTION

### Introduction

Sepsis is a complex life-threatening syndrome caused by a dysregulated host response to infection (1). The primary inflammatory response is often followed by a complex and protracted immunosuppressive response affecting both the innate and adaptive components of immunity. This post-septic immune suppression is associated with a higher risk of secondary infections, in the forefront of which are the intensive care unit (ICU)-acquired pneumonia (2).

However, a growing body of data suggest the existence of an important heterogeneity in the type and intensity of the immune response during post-septic immune suppression (3). Such heterogeneity is also retrieved at the patient level, sepsis from pulmonary origin being associated with a higher risk of subsequent ICU-acquired pneumonia when compared to sepsis from another origin (4). Furthermore, the recent COVID-19 pandemic has highlighted that the type of pathogen also influence the risk of ICU-acquired infection, the SARS-CoV-2 infected patients harboring a higher susceptibility to secondary pneumonia when compared to patients with flu or severe bacterial pneumonia (5).

While it is for example known from, e.g., WO 2010/082004, WO 2021/058919, WO 2022008827 or WO 2022008828, biomarkers for determining a risk of incidence of a care-related infection in patient, it is not only important in this context to determine appropriate diagnosis biomarkers, but also extremely crucial to develop tools that allow for patient stratification according to their risk of ICU-acquired infections, in particular in a context of secondary infections and at the earliest.

While in this setting, transcriptomic approaches are promising for relevant informing stratification strategies, this has been shown to be is mostly effective in the sepsis late phase. In a prospective cohort of septic patients, blood gene expression at the onset of ICU-acquired infections showed reduced expression of genes involved in gluconeogenesis and glycolysis (2). In ICU patients with ventilator-associated pneumonia (VAP), authors reported transcriptomic depression of genes involved in the immunological synapse in the blood. However, almost all studies investigate the transcriptomic response during the ICU-acquired infection rather than identify patients at risk to develop further infection. The recent development of multiplex molecular platforms such as the FilmArray^{®} System (bioMérieux) allows for rapid and reliable evaluation of the transcriptomic response of patients during nosocomial infections on the basis of the expressions of several genes involved in the pro- and anti-inflammatory response (6).

Present invention is aimed at proposing stratification tools, in particular based on the assessment of circulating mRNA of genes involved in the immune response. Inventors have presently highlighted that the development of such tools is constrained by the heterogeneity of septic patients. The aim of the study reported herein, on which the invention relies, is to evaluate the diagnosis performance of a transcriptomic score, which has, as a proof of concept, been performed between day 5 and day 7 after ICU admission on a range of patients. This transcriptomic score could identify a subgroup of critically patients who are likely to exhibit poor clinical outcomes, including higher rates of ICU-acquired infection, longer ICU length of stay and higher mortality. The invention therefore demonstrates the pertinence of the so-called transcriptomic score reported herein, initially based on a pragmatic combination of the outputs of immune-related genes detected with a prototype multiplex PCR tool, but defined through a particular rationale that makes it an unweighted score, in a strategy that proved to be conclusive, valuable, and pertinent with respect to other tools obtained by machine learning, for example.

The invention therefore relies on the experiments described herein, and proposes novel means and tools aimed at addressing any one or all of the above-mentioned problems or problems apparent from present description.

### DETAILED DESCRIPTION OF THE INVENTION

The invention relates to a method of determining, from an assayed sample or a population of assayed samples previously drawn from patients, a transcriptomic score (TScore) suitable for assessing a risk of adverse clinical outcome for patients in a clinical setting, said method comprising:
a. Measuring gene expression values on a patient sample or a population of samples previously obtained from patients, or if the method is computer-implemented retrieving such gene expression values, for at least two distinct genes selected among a predetermined set of genes,and
b. For each gene of the predetermined set of genes, and for each sample, comparing the measurements obtained or retrieved in step a. to predetermined threshold values identifying the assayed sample as pertaining to a patient presenting an infection or not presenting an infection, in particular an ICU-acquired infection, and/or determining on the basis of the measurements obtained or retrieved in step a., a receiver operator characteristics (ROC) curve aimed at representing the diagnostic ability of the gene expression value measured for said gene to identify the assayed sample as pertaining to a patient presenting an infection or not presenting an infection, in particular an ICU-acquired infection, and
c. For the assayed sample or each patient sample of the population of assayed samples, calculating a transcriptomic score where:
   i. For each gene of the predetermined set of genes analyzed individually, if the measured gene expression value matches the condition by which the sample is to be classified as a sample pertaining to a patient presenting an infection, in particular an ICU-acquired infection, assigning one point to the gene, and
   ii. Summing up the number of points obtained for all the genes of the predetermined set of genes, thereby obtaining a transcriptomic score for the assayed sample, and
   iii. Optionally, providing the transcriptomic score as an output value.

By "assessing a risk of adverse clinical outcome for patients in a clinical setting" it is meant that the definition of a transcriptomic score, which is at the core of present invention, is suited for, and, according to a particular embodiment is for, assessing the said risk. "Adverse clinical outcome" means that the clinical situation of the patient can be determined to be more serious or detrimental to the health of the patient at the time of the observation, or to be further aggravated at a later point of observation in time, with respect to the apparent clinical situation of the patient observed when the method has been carried out. Alternatively, it can be said that the method is for either assessing a risk of comorbidity in a patient, the sample of which has been subjected to a transcriptomic score analysis, or for assessing a risk of complication(s), complication(s) being defined as an unfavorable result of a disease, health condition, or treatment. According to a particular aspect, adverse clinical outcome can mean an increased rate/risk of occurrence of Intensive Care Unit (ICU) Acquired Infection(s) (Al), i.e., often abbreviated ICU-AI in present description, in the patient. An ICU-AI can be, according to a non-limitative list, ICU-acquired pneumonia, catheter-related bloodstream infection, urinary-tract infection. ICU-AI commonly encompass so-called "nosocomial infection", which are defined as infections patients acquire (or have probably acquired) while admitted to a health-care facility, and which generally develop after 48 hours, i.e., 48 hours or later, after admission. Common nosocomial infections are urinary tract infections, respiratory pneumonia, surgical site wound infections, bacteremia, gastrointestinal and skin infections. According to another aspect, an adverse clinical outcome can be a longer ICU length of stay. More generally, an adverse clinical outcome can be a longer stay in the hospital, which can also increase the chances of the patient to be put into contact with pathogens causing nosocomial infections. Examples of longer ICU length of stay are provided in present description and are readily and comprehensively available to a skilled person. According to another aspect, adverse clinical outcome can be seen as an higher ICU mortality, i.e., can be visualized by the death of the said patient.

Therefore, "adverse clinical outcome" generally means all clinical change of situation, which is unfavorable for the patient's health. According to a particular embodiment, "adverse clinical outcome" means the occurrence of an ICU-Al. Such occurrence can occur after the TScore described herein as been determined for the patient or be diagnosed for the patient after the TScore described herein as been determined in said patient.

"Patients in a clinical setting" generally means patients that are in a care facility, i.e., which have been admitted in care facility. According to more particular embodiments, patients in a clinical setting refer to patients which have been admitted in a reanimation unit, and/or an intensive or continuing care unit. According to a particular embodiment, applicable throughout present description, patients in a clinical setting are patients which have been admitted in an intensive care unit (ICU).

For the purpose of present method and in in order to provide the required gene expression values to the method, a patient's sample or patient samples is/are previously removed from a patient's body and then assayed, i.e., the invention is not directly carried out on a human body, i.e., it is an *in vitro* method as far as gene expression values are measured as part of the method. If the method is computer implemented, measured gene expression values can be provided as an input to the method, at the level of step a. of the method as described above. The expression "retrieving gene expression values" means that gene expression values are provided as input data to the method to be carried out by a computer, if implemented as such.

With regards to step a. of the method of determining a transcriptomic score (TScore) described above and herein, the "patient sample or a population of samples previously obtained from patients", can be from patients as defined above. According to a particular embodiment, the "patient sample or a population of samples previously obtained from patients" is/are from patients admitted in an Intensive Care Unit (ICU).

Of note, since the patients of the method of determining a transcriptomic score (TScore) described above and herein are patients admitted in a care facility, in particular an intensive care unit (ICU), such patients can have underlying pathologies or conditions, including under severe forms thereof. As stated in the examples, and according to a non limitative list, patients can be susceptible of having or be diagnosed with sepsis at different stages, trauma, including severe trauma, or burns. Examples are provided in the experimental section, which are however not limitative of the conditions that be encountered in intensive care units. A transcriptomic score (TScore) can be defined regardless of the initial condition of the patient, and regardless of the fact that the patient is currently undergoing therapy for the condition, such as antibiotics.

According to a specific and non-limitative embodiment, the assayed biological sample(s) is (are) drawn from patient(s) susceptible to be suffering of, or susceptible of being diagnosed with, or suffering from sepsis.

With regards to step a. of the method of determining a transcriptomic score (TScore) described above and herein, and according to a particular embodiment, a gene expression value is measured through mRNA expression levels. In a particular embodiment, mRNA expression levels are measured in particular through a multiplex assay involving a plurality of assayed genes. According to a different particular embodiment, gene expression values are measured by a molecular method, such as an amplification method, by sequencing (e.g. Next Generation Sequencing), or by hybridization methods (e.g. hybridization microchips, NanoString^{®} nCounter^{®} methods). All these methods are well known by the skilled person.

The skilled person will readily understand that in step a., "measuring gene expression values on a sample or a population of samples previously obtained from patients" is preferably carried out at a given time, in particular a time that is identical for all assayed genes, the expression values of which is measured. Use of a multiplex assay favors such a configuration.

According to a specific and non-limitative embodiment, the assayed biological sample(s) is (are) drawn from patient(s) admitted in an Intensive Care Unit (ICU), preferably between days 5 and 7 of the ICU stay.

According to a particular embodiment, a gene expression value is measured by RT-PCR, in particular RT-qPCR, more particularly nested RT-qPCR, especially a multiplex RT-PCR or RT-qPCR or nested RT-qPCR, for example using the FilmArray^{®} technology (bioMérieux) or Biomark^{™} platform (Fluidigm). The skilled person can readily implement such methods on the basis of the guidance provided in the field and herein. For example, the skilled person can easily find the mRNA sequences in the NCBI or Ensembl databases and can design the sequences of primers or pairs of primer necessary for measuring mRNA expression levels using commonly available tools such as Geneious or Primer 3.

According to a particular embodiment, the assayed biological sample(s) is (are) blood sample(s), preferably total blood sample(s).

In the context of step a. of the method of determining a transcriptomic score (TScore) described above and herein, gene expression values are measured or the measurements are retrieved for at least two distinct genes selected among a predetermined set of genes.

As detailed in the experimental section herein, it is an asset of present method that the considered genes are preferably known to be independent or simply deemed to be independent, for the purpose of determining a TScore. Guidance is provided in the experimental section regarding the meaning of such an established or putative independence. The skilled person can therefore readily determine a subset of genes that can be included in the method, for instance on the basis of biomarkers, the pertinence of which is assessed in the literature, or on the basis of a postulate guided by his/her knowledge. Indeed, for performance of the method, it is not required that the genes be actually independent, since this artefact enables to grasp the heterogeneity/complexity of patient's characteristics in clinical settings and turn it as an advantage.

According to a particular embodiment, the genes of the predetermined set of genes of step a. of the method of determining a transcriptomic score (TScore) described above and herein are selected among: *ADGRE3* (Adhesion G protein-coupled receptor E3), *ARL14EP* (Ribosylation factor like GTPase 14 effector protein), *BPGM* (Biphosphoglycerate mutase), *C3AR1* (Complement C3a receptor 1), *CCN81IP1* (Cyclin B1 interacting protein 1), *CD177* (CD177 molecule), CD274 (CD274 molecule), *CD3D* (CD3d molecule), *CD74* (CD74 molecule), *CIITA* (Class II major histocompatibility complex transativator), *CTLA4* (Cytotoxic T-lymphocyte associated protein 4), *CX3CR1* (C-X3-C motif chemokine receptor 1), *GNLY* (Granulysin), IFNgamma (interferon gamma), *IL10* (Interleukin 10), *IL1R2* (Interleukin 1 receptor 2), *IL1RN* (Interleukin 1 receptor antagonist), *IL7R* (Interleukin 7 receptor), *IP10*/*CXCL10* (Interferon gamma induced protein 10), *MDC1* (Mediator of DNA damage checkpoint 1), *OAS2* (2'-5'-oligoadenylate synthetase 2), *S100A9* (S100 calcium bonding protein A9), *TAP2* (Transporter 2, ATP binding cassette subfamily B member), *TDRD9* (Tudor domain containing 9), *TNF* (Tumor necrosis factor) and *ZAP70* (Zeta chain of T cell receptor associated protein kinase 70). Chromosomic location and sequences of the predetermined set of gene can be found by the skilled in NCBI or Ensembl databases.

According to distinct particular embodiments, the number of assayed genes is at least 2, 3, 4, 5, 6, 7, 8, 9, 10, 11, 12, 13, 14, 15, 16, 17, 18, 19, 20, 21, 22, 23, 24, 25 or 26. According to distinct particular embodiments, the number of assayed genes is 2, 3, 4, 5, 6, 7, 8, 9, 10, 11, 12, 13, 14, 15, 16, 17, 18, 19, 20, 21, 22, 23, 24, 25 or 26. According to other distinct particular embodiments, the number of assayed genes is at least 2, 3, 4, 5, 6, 7 or 8, in particular is any of 2, 3, 4, 5, 6, 7 or 8.

According to step b. of the method of determining a transcriptomic score (TScore) described above and herein, and for each gene of the predetermined set of genes, and for each sample, it is proposed to compare the measurements obtained or retrieved in step a. to predetermined threshold values identifying the assayed sample as pertaining to a patient presenting an infection or not presenting an infection, in particular an ICU-acquired infection, and/or to determine a receiver operator characteristics (ROC) curve aimed at representing the diagnostic ability of the gene expression value measured for said gene to identify the assayed sample as pertaining to a patient presenting an infection or not presenting an infection, in particular an ICU-acquired infection.

By "comparing the measurements obtained or retrieved in step a. to predetermined threshold values", it is meant comparing the values measured with threshold values for discriminating between samples pertaining to a patient presenting an infection, in particular an ICU-acquired infection, versus samples pertaining to patient not presenting an infection, in particular an ICU-acquired infection.

The expression "sample pertaining to a patient not presenting an infection" can for deemed synonymous to the expression "sample not pertaining to a patient presenting an infection" and according to this rule can be used without distinction within the meaning of the present invention.

By "determining a receiver operator characteristics (ROC) curve" it is meant either calculating such a curve, according to the knowledge of the skilled person in the field, or drawing or plotting such a curve, the latter in particular when the method is computer-implemented. A ROC curve is a well-known graphical method of displaying the discriminatory accuracy of a marker (diagnostic test) for distinguishing between two populations. It can be used to investigate the effectiveness of diagnostic markers in distinguishing between diseased and healthy individuals. Generally, a person is assessed as diseased (positive) if the tested marker value is greater than a given, predetermined, threshold value, otherwise the subject is diagnosed as healthy (negative). The accuracy of any given threshold value can be measured by the probability of a true positive (sensitivity) and the probability of a true negative (specificity). A ROC curve is a plot of the sensitivity (Se(c)) versus 1-specificity (1-Sp(c)) over all possible threshold values (c) for a considered marked. In present invention, the markers are the expression levels of the genes defined according to any embodiment described herein and the diagnosis sought is the discrimination between samples pertaining to patients presenting an infection, in particular an ICU-acquired infection, versus samples pertaining to patients not presenting an infection, in particular an ICU-acquired infection.

On the one hand threshold levels can commonly be determined by considering data (gene expression levels) obtained or known for healthy individuals, i.e., not presenting an infection, in particular an ICU-acquired infection, as it is known for any diagnostic test. Such levels can be provided to the method of the invention as predetermined threshold values.

On the other hand, for the purpose of obtaining a training testing and associated validation data, such as a reference transcriptomic score and decision values such as an optimal threshold value, which will be discussed hereafter, or even, according to a particular embodiment, threshold values for the gene expression levels in the context of a training testing, the measurements or retrieved measured data of step a. of the method of determining a transcriptomic score (TScore) described above and herein are provided on a population of assayed samples, so that ROC curves can be drawn in step b.

In such a situation, the patients of the training set, from which samples are drawn prior to implementation of the method of the invention, encompass both patients presenting an infection at the time of the measurement, and patients not presenting an infection at the time of the measurement, i.e., encompasses patients for which it is known whether they had an infection, especially an infection occurring within the timeframe discussed herein after their admission in a care facility (according to the definitions provided above), more specifically patients for which it is known whether they had at least one episode of ICU-acquired infection (ICU-AI), especially since their admission in an Intensive Care Unit (ICU). The experimental section herein provides guidance in this respect.

Conversely, when a sample is tested against predetermined threshold values, e.g., for classification purpose, then a single sample can be assayed.

When a method of determining a transcriptomic score (TScore) described above and herein is carried out on a population of assayed samples previously drawn from patients for which it is known for a part of the samples whether they have been drawn from patients that have presented at least one episode of infection since their admission in a care unit, notably their admission in an ICU, such a method can further comprises, after the step b. of the method of determining a transcriptomic score (TScore) described above and herein, a step of further selecting, within the predetermined analyzed set of genes, genes for which the AUC value of the ROC curves drawn in step b. are at least 0.70.

According to a particular embodiment, the AUC value of the ROC curves drawn in step b. is superior or equal to: 0.70, 0.71, 0.72, 0.73, 0.74, 0.75, 0.76, 0.77, 0.78, 0.79, 0.80, 0.81, 0.82, 0.83, 0.84, 0.85, 0.86, 0.87, 0.88, 0.89, 0.90, 0.92, 0.93, 0.94, 0.95, 0.96, 0.97, 0.98, 0.99, or is 1.

The Area Under the Curve (AUC) is commonly known in the field and is the measure of the ability of a classifier to distinguish between classes; it can be used as a summary of the ROC curve. The higher the AUC, the better the performance of the model at distinguishing between the positive and negative classes.

For instance, such an implementation allows to refine the predetermined set of genes that is used for analysis. According to a particular embodiment, the updated predetermined set of genes can be provided as an output value to the method.

As it is the practice of the skilled person in the art, an AUC value comes with peculiar sensitivity and specificity values, which are associated with the considered test and therefore known to the skilled person, as long as the ROC curve is available to the skilled person.

According to a particular embodiment, the predetermined set of genes used in a method of the invention as described herein, before or after selecting, if carried out, genes for which the AUC value complies with the above requirements, encompasses at least 4 genes, preferably at least 6 or 8 genes, in particular encompasses 6 or 8 genes. According to a particular embodiment, the predetermined set of genes, refined or not, used in a method of the invention consists in the following genes: C3AR1, CD177, CX3CR1, IFNgamma, IL1R2, S100A9, TDRD9 and ZAP70. According to a particular embodiment, the predetermined set of genes, refined or not, used in a method of the invention consists in the following genes: C3AR1, CD177, CX3CR1, IFNgamma, IL1R2 and S100A9.

When a method of determining a transcriptomic score (TScore) described above and herein is carried out on a population of assayed samples previously drawn from patients for which it is known for a part of the samples whether they have been drawn from patients that have presented at least one episode of infection since their admission in a care unit, notably their admission in an ICU, such a method can further comprises, after the step b. of the method of determining a transcriptomic score (TScore) described above and herein, and if relevant after determination of a refined set of genes, a step of determining a threshold value for each gene of the predetermined set of genes that is analyzed (refined or not), in particular through calculation of the Youden Index on the ROC curve determined in step b. described herein, more particularly wherein the threshold value is the value for which the Youden Index is maximized, and optionally providing the threshold value for the gene as an output value.

Such an embodiment provides for the situation where threshold values are defined on the basis of the measurement values obtained on transcript of, for example, a training sample, and not by reference to threshold values obtainable separately, provided as predetermined threshold values to the method, e.g., on the basis of data obtained on healthy individuals which are deemed healthy for any medical condition (such as healthy volunteers).

The Youden Index, also termed "J Statistic" in the literature, is a well-known indicator that represents a summary measurement of the receiver operating characteristic (ROC) curve for the accuracy of a diagnostic test (see, e.g., Youden WJ. Index for rating diagnostic tests. Cancer. 1950; 3(1):32-35). It can be defined as follows: Youden Index = Sensitivity + Specificity - 1, or Youden Index = [TP / (TP + FN)] + [TN / (FP + TN)] - 1 where TP = True Positives, TN = True Negatives, FP = False Positives, FN = False Negatives. Youden Index ranges between 0 and 1, with 0 values indicating that a diagnostic test gives the same proportion of positive results for groups with and without the disease. A value of 1 indicates that there are no false positives or false negatives. The Youden Index can be used in conjunction with a receiver operating characteristic (ROC) analysis, where the index is defined for all points of the ROC curve. The maximal value of the index can then be used as a cut-off for numerical diagnostic tests, as suggested in the embodiment detailed in preceding paragraphs.

According to a particular embodiment where gene expressions values are measured through mRNA expression levels, preferably by RT-qPCR and in particular according to the protocol described in the experimental section of present description, and the genes of the predetermined set of genes are selected among: C3AR1, CD177, CX3CR1, IFNgamma, IL1R2, S100A9, TDRD9 and ZAP70, it can for example be determined that the predetermined threshold value of step b. of a method of determining a transcriptomic score (TScore) described above and herein are, for each gene respectively, as follows:
- C3AR1: 0.360
- CD177: 2.867
- CX3CR1: -1.679
- IFNgamma: -6.601
- IL1R2: 3.894
- S100A9: -0.189
- TDRD9: 1.923
- ZAP70: 2.482

According to a particular embodiment where gene expressions values are measured through mRNA expression levels, preferably by RT-qPCR and in particular according to the protocol described in the experimental section of present description, and the genes of the predetermined set of genes are selected among: C3AR1, CD177, CX3CR1, IFNgamma, IL1R2 and S100A9, it can for example be determined that the predetermined threshold value of step b. of a method of determining a transcriptomic score (TScore) described above and herein are, for each gene respectively, as follows:
- C3AR1: 0.371
- CD177: 3.199
- CX3CR1: -1.678
- IFNgamma: -5.172
- IL1 R2: 3.912
- S100A9: 0.294

According to conventions commonly used in the field, the "-" sign reflects the fact that some genes can have their expression decreased with respect to a reference pool of samples drawn from patients not presenting an infection, in particular an ICU-Acquired Infection.

One can observe that it is within the knowledge of the skilled person to readily adapt the comparison made with respect to a threshold value taking into account whether the expression of the gene is increased or decreased with respect to a reference pool of samples as stated above. Furthermore, the skilled person can readily compose with the margin of error associated with the instruments used for measurement mRNA expression levels, whatever the actual method of measurement is used.

According to step c. of the method of determining a transcriptomic score (TScore) described above and herein, a transcriptomic score is calculated, as follows: for the assayed sample or each patient sample of the population of assayed samples, a transcriptomic score is calculated where:
i. For each gene of the predetermined set of genes analyzed individually, if the measured gene expression value matches the condition by which the sample is to be classified as a sample pertaining to a patient presenting an infection, in particular an ICU-acquired infection, assigning one point to the gene, and
ii. Summing up the number of points obtained for all the genes of the predetermined set of genes, thereby obtaining a transcriptomic score for the assayed sample, and
iii. Optionally, providing the transcriptomic score as an output value.

By "measured gene expression value matches the condition by which the sample is to be classified as a sample pertaining to a patient presenting an infection", it is meant that the measured gene expression value is compared to a threshold value, predetermined and provided or obtained through a receiver operator characteristics (ROC) curve and use of a Youden Index, including by taking into consideration whether the gene expression is increased or decreased in patients for the considered diagnosis (presence of an infection, in particular an ICU-AI) with respect to patients healthy for the considered diagnosis.

It will be understood that the maximal value of a transcriptomic score depends on the number of genes on the basis of which it is built. For example, if the transcriptomic score is built using the expression values of 8 genes, then the TScore can range from 0 to 8. If the transcriptomic score is built using the expression values of 6 genes, then the TScore can range from 0 to 6. Guidance can be found in the experimental section.

Further so-called "associated validation data" can be determined for a TScore of present invention. For example, when carried out on a population of assayed samples previously drawn from patients for which it is known for a part of the samples whether they have been drawn from patients that have presented at least one episode of infection since their admission in a care unit, notably their admission in an ICU, a method of the invention can further comprise determining an optimal threshold value which is associated to the transcriptomic score calculated in step c.ii of the method of determining a TScore described herein, through calculation of the Youden Index of the said transcriptomic score, in particular wherein the optimal threshold value is the value for which the Youden Index is maximized. According to such an embodiment, such an optimal threshold value (for a TScore) is be calculated on the basis of the ROC curve of a transcriptomic score. The same consideration as above apply regarding the Youden Index. Such an optimal threshold value or the corresponding Youden Index or both can be provided as output value(s) to the method.

Definition of an optimal threshold value for a TScore allows for an easier use of such a TScore as a diagnosis tool, with respect to the examined condition, i.e., the assessment of a risk of adverse clinical outcome for patients in a clinical setting, according to any definition provided herein, as it will be seen hereafter.

The invention also relates to a method to classify a sample previously drawn from a patient, in particular previously drawn from a patient admitted in an Intensive Care Unit (ICU), in a group (of samples) reflecting a risk of adverse clinical outcome in a clinical setting for patients in such a setting, said method comprising the steps of:
a. Carrying out a method of determining a transcriptomic score (TScore) according to any embodiment described herein on a sample previously drawn from a patient (that is to be classified) in order to calculate a transcriptomic score (for said sample), and
b. Comparing the calculated transcriptomic score of step a. of the present classification method to a predetermined threshold value, in particular a predetermined threshold value that is an optimal threshold value determined through calculation of a Youden index on a transcriptomic score obtained on a reference or training population of assayed samples, for example comparing the calculated transcriptomic score of step a. of the classification method to an optimal threshold value which a been predetermined with a method as separately defined in the above paragraphs, and
c. From the comparison of step a. of the present classification method, assigning the sample drawn from the patient to a group of samples reflecting a risk of adverse clinical outcome in a clinical setting, and
d. Optionally, providing the classification result obtained in step c. of the present classification method as an output value.

According to a particular embodiment, care is taken that the experimental conditions for the measurements carried out on a sample to be classified, are similar if not identical to the experimental conditions that have been used to define the predetermined threshold value, especially an optimal threshold value, used as reference in step b. of the classification method. In particular, care can be taken that the same genes or the same methods of measurement are used, in order to allow for a reliable comparison. To this end, the experimental conditions of the reference or training population of assayed samples referred to in step b. of the classification method can be used.

For example, as illustrated in the experimental section herein, the determination of a transcriptomic score where the following genes: C3AR1, CD177, CX3CR1, IFNgamma, IL1R2, S100A9, TDRD9 and ZAP70 have been assayed, allowed for determining that presence of a risk of adverse clinical outcome when the transcriptomic score calculated for the patient assayed sample is equal or greater than 3 (the optimal threshold value for the TScore being set at 2). The same applies when, as described in the experimental section herein, the transcriptomic score encompasses the following genes: C3AR1, CD177, CX3CR1, IFNgamma, IL1R2 and S100A9.

It follows that assigning the sample drawn from the patient to a group of samples reflecting a risk of adverse clinical outcome in a clinical setting means classifying the sample within either a subgroup of samples of patients with a so-called higher risk of adverse clinical outcome in a clinical setting, or a subgroup of samples of patients with so-called a lower risk of adverse clinical outcome in a clinical setting, with the definitions for "adverse clinical outcome" being as detailed above and herein. The use of the adjectives "high" and "low" defines, in the present context, the fact of being above or below the defined threshold deemed to provide a pertinent cut-line for discriminating the samples, in particular on the basis of the fact that the patients discriminated as such may have specific immune dysfunction backgrounds. The use of the adjectives "high" and "low" with respect to the threshold discussed in the previous sentence also means that the method comes with a cut-line that is deemed pertinent in association with parameters such as the sensitivity or specificity of the considered test or method, given the context in which it is to be used.

According to a particular embodiment, a so-called "classification" method can also be termed a "stratification" method, a term which is commonly used in the field.

According to a particular embodiment, the methods described herein are computer-implemented, i.e., is partly or fully carried out by computer means. In such a context, it is provided for the possibility of inputting or outputting data fed or obtained by the disclosed methods, for example in variables to be stored in a memory, or as parameters to be displaying on an output visualization system.

The invention also relates to a method to identify a patient at risk of adverse clinical outcome in a clinical setting, comprising carrying out a method of classification (or stratification) as disclosed in any embodiment herein, on a sample previously drawn from a patient, and identifying the patient as having a risk of adverse clinical outcome in a clinical setting on the basis of the basis of the group to which the sample has been assigned to. Conclusion of risk follows to conclusion that can be attached to the classification of the assayed sample in the so-called group with higher risk or lower risk of adverse clinical outcome in a clinical setting. Definitions of "adverse clinical outcome" are as provided elsewhere in present description, according to any disclosed embodiment. Accordingly, such a method can be qualified as a diagnosis method as far as the defined risk applies to a health condition framed by the definition of a disease. Otherwise, the method of the invention is a method of classification on the basis of a biological parameter.

According to particular embodiments, an adverse clinical outcome is:
- the occurrence of complications, such as a care unit-acquired infection, in particular the occurrence of an intensive care unit acquired infection (ICU-AI), notably a nosocomial infection, and/or
- a prolonged stay in care unit (in particular an ICU), and/or
- death.

According to preferred embodiment, the adverse clinical outcome is the occurrence of complications, preferably a care unit-acquired infection, in particular the occurrence of an intensive care unit acquired infection (ICU-AI), notably a nosocomial infection.

The invention therefore also qualifies for a method for determining the survival rate of a patient in a clinical setting, according to any one of the definitions provided for this term in present description.

The invention also relates to a method as disclosed in any embodiment herein, which is for:
a. *in vitro* or *ex vivo* diagnosing or prognosticating whether a patient is at risk of adverse clinical outcome in a clinical setting, in particular whether the patient is at risk of acquiring an intensive care unit acquired infection, in particular a nosocomial infection, and/or
b. *in vitro* or *ex vivo* monitoring the evolution of the health status of a patient over time, in particular when the method is carried out at several distinct points of time on several distinct samples drawn from the patient, the monitoring of the evolution consisting in comparing the conclusion reached at the different sampling times, and/or
c. *in vitro* or *ex vivo* classifying the patient as pertaining to a group displaying immune alterations in circulating cells, and/or *in vitro* or *ex vivo* diagnosing or prognosticating whether a patient has an immune dysfunction background.

Classifying the patient as pertaining to a group displaying immune alterations in circulating cells amounts to screening (through an in vitro or ex vivo sample assay) the immune status of a patient, for the purpose that the patient may, depending upon its immune background, be more or less responsive to some immune modulating treatment or drugs. For example, as shown in the experimental section, inventors found that the patients classified as "high-risk" for further infections had biological features of immune suppression, with low levels of monocytic HLA-DR and high levels of the anti-inflammatory cytokine IL-10.

The invention also relates to an *in vitro* or *ex vivo* method of screening whether a drug has the capacity to alleviate an adverse clinical outcome, as defined in any one of the embodiments described herein, in a patient, the method comprising the steps of:
- Determining whether a patient is at risk of adverse clinical outcome in a clinical setting, by carrying out, at a first point in time, a method as disclosed in any embodiment herein on a sample previously drawn from the patient, and
- If the patient is determined to be at risk of adverse clinical outcome in a clinical setting, reiterating, at a second point in time placed after the first point in time and after treatment of the said patient with a drug deemed to have the capacity to alleviate the adverse clinical outcome, the carrying out of a method as disclosed in any embodiment herein (preferably the same method as the method used at the first point in time) on a sample previously drawn from such a patient at the second point in time that is later than the first point in time, and
- Optionally, concluding about the capacity of the drug to alleviate an adverse clinical outcome.

Conclusion can readily be made by observation of any change made apparent or investigated after treatment.

By "drug deemed to have the capacity to alleviate the adverse clinical outcome" it is for example meant a drug which can be beneficial to a patient classified as a "high-risk" patient according to the present invention.

Throughout the present description it is observed that if a method as described in any embodiment detailed herein, is computer-implemented, then it can be carried out, partly or fully, by computer means. Accordingly, the invention also relates to a computer-implemented method that includes the steps of a method according to the invention, according to any embodiment disclosed in present application, in particular a method or one or more steps are carried out by a computer or computer means or means commanded by a computer.

To this end, the invention also provides for a data processing apparatus comprising:
a. means for carrying out a computer-implemented method according to the invention, especially means for retrieving gene expression values and/or means for inputting reference values, and/or means for determining ROC curves and/or means for determining if decision conditions are met or calculating decision conditions and/or means for calculating a transcriptomic score, and/or means to provide output values, optionally means for measuring gene expression values on a biological sample previously drawn from a patient, when said means are carried out or driven by a computer, or
b. comprising a processor adapted to/configured to perform a computer-implemented method according to the invention, especially a processor adapted to/configured to perform the steps of a computer-implemented method according to the invention.

According to a particular embodiment, such a data processing apparatus comprises:
a. an input interface to receive, as defined in step a. of the method of determination of a TScore detailed in any embodiment herein, levels of gene expression values, in particular the levels of at least two genes as defined herein,
b. a memory for storing at least instructions of a computer program comprising instructions which, when the program is executed by a computer or processor, cause the computer to carry out a method according to claim 16, optionally a memory for storing data and decision rules,
c. a processor accessing to the memory for reading the aforesaid instructions and executing a computer-implemented method according to the invention,
d. an output interface to provide output values, in particular the output values defined in any embodiment described in present description, and/or to output the conclusion reached for any method claims as described herein.

According to another aspect, the invention also relates to a computer or computer system configured to perform the actions recited in the steps of a computer-implemented method according to the invention, according to any embodiment disclosed in present application.

The invention also relates to a computer program comprising instructions which, when the program is executed by a computer or processor, cause the computer to carry out a computer-implemented method according to the invention.

The invention also relates to a computer-readable medium, in particular a computer-readable non-transient recording medium, having stored thereon a computer program as disclosed in any embodiment herein, especially to implement a computer-implemented method according to the invention, when the computer program is executed by a computer or processor. According to another aspect, such a computer readable medium which may be a computer-readable non-transient recording medium, is a computer readable medium that configures a computer to perform the actions recited in the steps of a computer-implemented method according to the invention, according to any embodiment disclosed in present application.

The invention also relates to the use of a kit comprising means for amplifying and/or detecting gene expression values for at least two distinct genes selected among the genes defined in any embodiment herein, in particular genes selected among: C3AR1, CD177, CD3D, CD74, CIITA, CTLA4, CX3CR1, IFNgamma, IL1R2, TAP2, S100A9 TDRD9 and ZAP70, for carrying out a method as disclosed in present description, especially by measuring the gene expression values of at least two distinct genes selected among the genes recited above in a biological sample previously removed from a human patient, in particular a human patient in a clinical settings, which has been admitted in an hospital, especially a human patient susceptible of being diagnosed as being at risk of sepsis or a patient susceptible of suffering of condition(s) than can evolve in a sepsis event.

According to a particular embodiment, a kit suitable for carrying out a method of the invention as defined in any embodiment disclosed herein, comprises:
- at least one, in particular two, pair of specific oligonucleotide primers specific for hybridization with mDNA coding for, respectively, two or more of C3AR1, CD177, CD3D, CD74, CIITA, CTLA4, CX3CR1, IFNgamma, IL1R2, TAP2, S100A9 TDRD9 and ZAP70, and, optionally, one or several of the following reagents,
- nucleotides (e.g. dATP, dCTP, dGTP, dUTP),
- a reverse transcriptase,
- a DNA polymerase, in particular a thermostable DNA polymerase, such as a Taq DNA Polymerase,
- at least one dye for staining nucleic acids, in particular a dye detectable in a real-time PCT equipment,
- optionally, a buffer solution,
- optionally, reagents necessary for the hybridization of the primers to their targets,
- optionally, a reference dye and,
- optionally, a data processing apparatus, in particular as detailed in any embodiment described herein,
- optionally, a computer program, in particular as detailed in any embodiment described herein,
- optionally, a computer-readable medium, in particular as detailed in any embodiment described herein,
- optionally, a notice providing instructions for use and expected values for interpretation of results.

The term "comprising" as used herein, which is synonymous with "including" or "containing", is openended, and does not exclude additional, unrecited element(s), ingredient(s) or method step(s), whereas the term "consisting of" is a closed term, which excludes any additional element, step, or ingredient which is not explicitly recited.

The term "essentially consisting of" is a partially open term, which does not exclude additional, unrecited element(s), step(s), or ingredient(s), as long as these additional element(s), step(s) or ingredient(s) do not materially affect the basic and novel properties of the application.

The term "comprising" (or "comprise(s)") hence includes the term "consisting of" ("consist(s) of"), as well as the term "essentially consisting of" ("essentially consist(s) of"). Accordingly, the term "comprising" (or "comprise(s)") is, in the present application, meant as more particularly encompassing the term "consisting of" ("consist(s) of"), and the term "essentially consisting of" ("essentially consist(s) of").

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated.

Each of the relevant disclosures of all references cited herein is specifically incorporated by reference.

The features described here-above and other features of the invention will be apparent when reading the examples and the figures, which illustrate the experiments conducted by the inventors, in complement to the features and definitions given in the present description. The following examples are offered by way of illustration. The examples are however not limitative with respect to the described invention.

### LEGEND OF THE FIGURES

**Figure 1****. Characteristics and methods for the discovery cohort**
   **A:** Flow-chart diagram of the discovery cohort, **B:** Scheme depicting the method used to identify candidate genes.
**Figure 2****. Gene's identification process in the discovery cohort**
   **A:** ROC (Receiver Operating Characteristics) curves with AUC (area under the curve) upper than 0.70 of several genes (C3AR1: Complement C3a Receptor 1, CD177 : CD177 molecule, CX3CR1: C-X3-C motif chemokine receptor 1, IFNy: Interferon gamma, IL1R2: Interleukin 1 receptor 2, S100A9: S100 calcium binding protein A9, TDRD9: Tudor domain containing 9, ZAP70: Zeta chain of T cell receptor associated protin kinase 70), **B:** Normalized RNA values for identified genes in patient with (red dots) or without (green) ICU-acquired infections (ICU-AI).

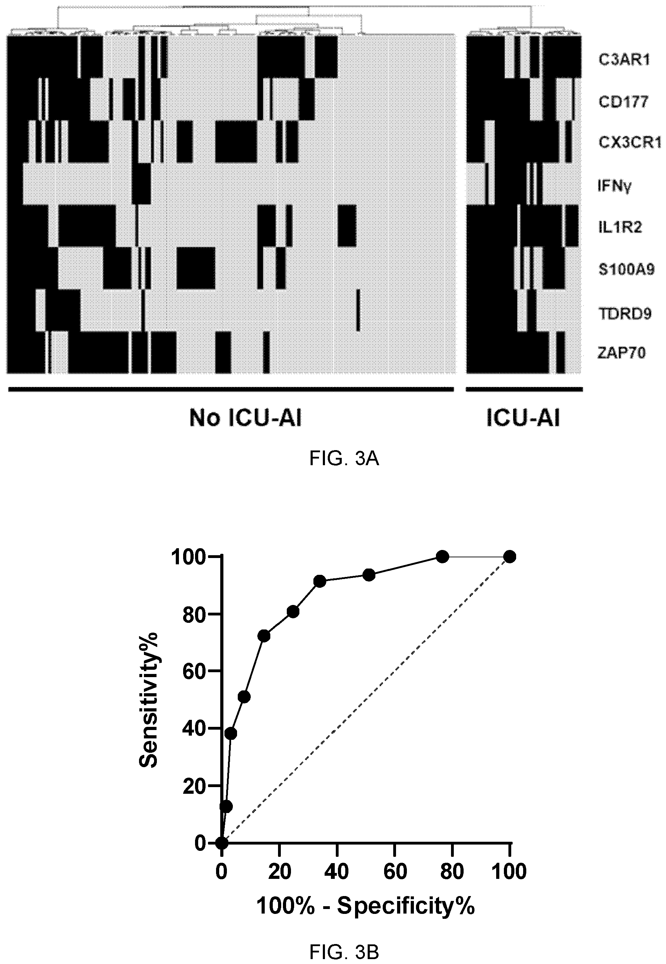

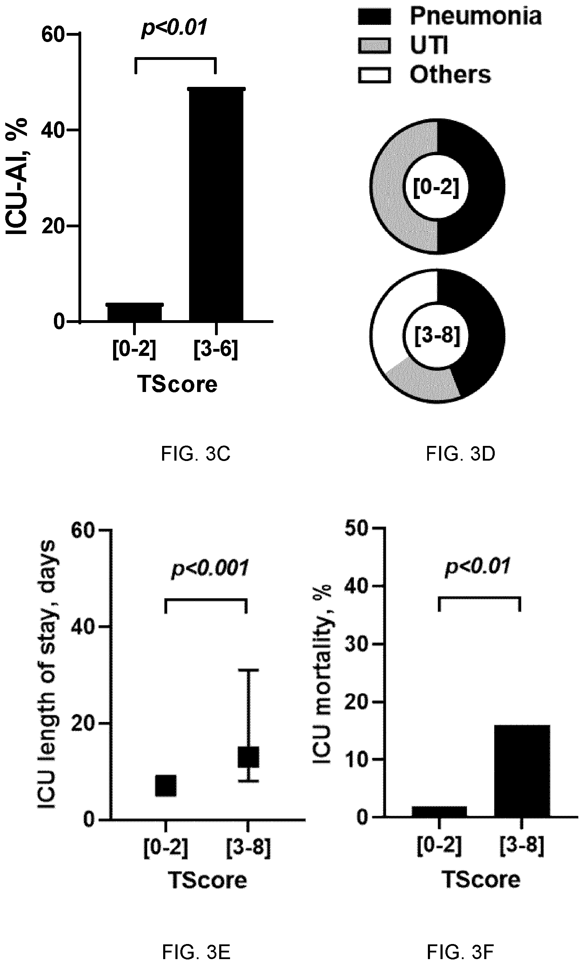

**Figure 3****. Clinical outcomes of high risk and low risk patients in the discovery cohort using the TScore A:** Heatmap of unsupervised hierarchical clustering in patients with or without ICU-acquired infections using individual gene score, **B**: ROC (Receiver Operating Characteristics) curve with AUC (area under the curve) value of the ability of the Tscore obtained at day 5-7 to distinguish between patients that will develop or not at least an ICU-acquired infection during their ICU stay, **C:** Intensive care unit acquired infection (ICU-AI) proportion in patients with a Tscore between 0 and 2 (low risk) or upper or equal to 3 (high risk), **D:** Distribution of the type of ICU-AI in patients with a Tscore between 0 and 2 (low risk) or upper or equal to 3 (high risk) (UTI: urinary tract infections), **E:** Median and interquartile duration of intensive care unit length of stay in patients with a Tscore between 0 and 2 (low risk) or upper or equal to 3 (high risk), **F:** intensive care unit mortality rate in patients with a Tscore between 0 and 2 (low risk) or upper or equal to 3 (high risk)

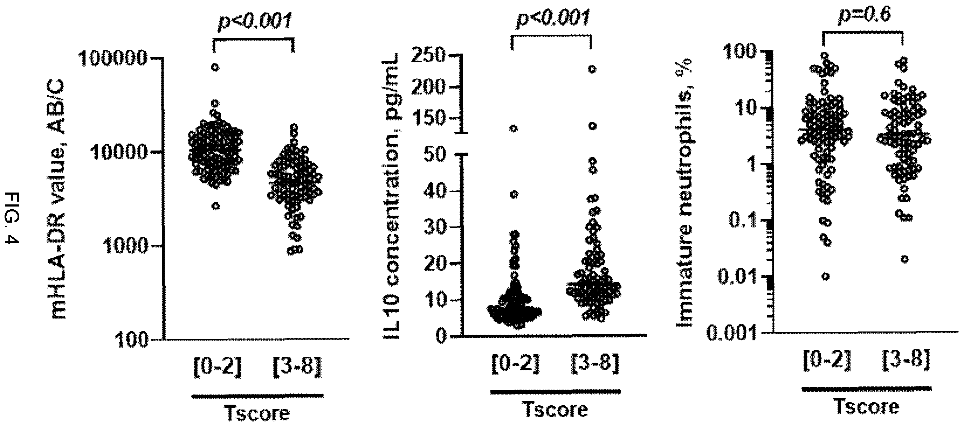

**Figure 4****. Statistical and immunological validity of the TScore in the discovery cohort**
   Level of expression of several immunological parameters (monocytic HLA-DR, IL-10 and immature neutrophils proportion) measured in blood of patients with a Tscore between 0 and 2 (low risk) or upper or equal to 3 (high risk)

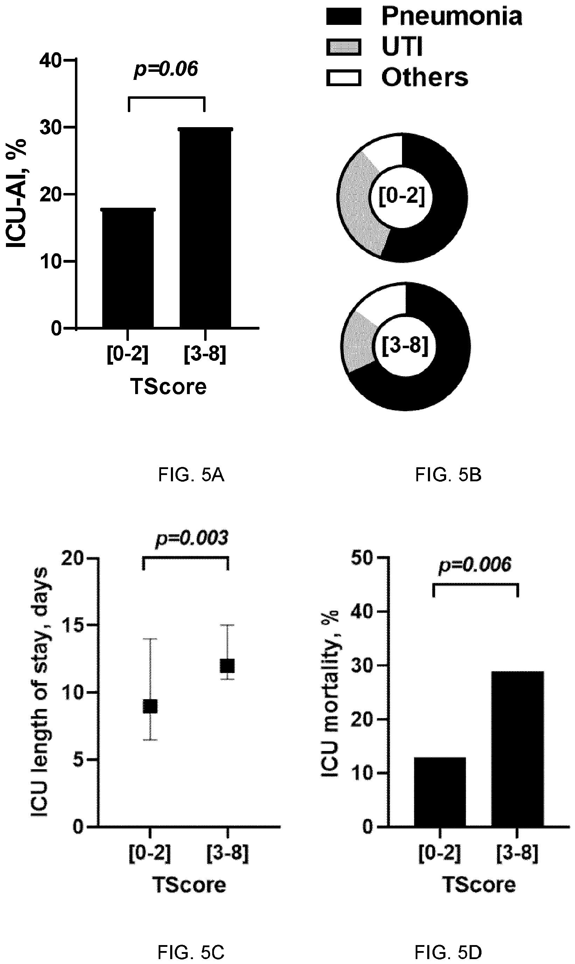

**Figure 5****. Clinical outcomes of high risk and low risk patients in the validation cohort using the TScore.**
   **A:** Intensive care unit acquired infection (ICU-AI) proportion in patients with a Tscore between 0 and 2 (low risk) or upper or equal to 3 (high risk), **B**: Distribution of the type of ICU-AI in patients with a Tscore between 0 and 2 (low risk) or upper or equal to 3 (high risk) (UTI: urinary tract infections), **C:** Median and interquartile duration of intensive care unit length of stay in patients with a Tscore between 0 and 2 (low risk) or upper or equal to 3 (high risk), **D:** intensive care unit mortality rate in patients with a Tscore between 0 and 2 (low risk) or upper or equal to 3 (high risk).

### EXAMPLES

### METHODS

**Summary:** As training cohort, the inventors used the gene expression dataset obtained from 176 critically ill patients enrolled in the REALISM study (NCT02638779) with various etiologies and still hospitalized in intensive care unit (ICU) at day 5-7. Based on the performances of each gene taken independently to identify patients presenting ICU-acquired infections (ICU-AI) after day 5-7, the inventors built an unweighted score assuming the independence of each gene. The inventors then determined the performances of this score to identify a subgroup of patients at high-risk to develop ICU-AI, and both ICU length of stay and mortality of this high-risk group were assessed. Finally, they validated the effectiveness of this score in a retrospective cohort of 257 septic patients.

### Patients and setting

In the training cohort, the patients and the data are abstracted from the previously published REALISM (REAnimation Low Immune Status Marker) cohort study (7). Briefly, this monocenter observational cohort study included critically ill patients with sepsis, trauma and burn patients. Inclusion criteria were: patients aged > 18 years, clinical diagnosis of sepsis as defined by the SEPSIS-3 consensus guidelines (1), severe trauma with injury severity score (ISS) > 15 or total burned surface area >30%. Exclusion criteria were any of the following: presence of a preexistent condition or treatment that could influence patients' immune status, pregnancy, institutionalized patients, inability to obtain informed consent. Whereas data and biological samples were collected at day 1 or 2 (D1-2), D3 or 4 (D3-4) and D5, D6 or D7 (D5-7), we focused on day 5-7. Longitudinal follow-up was performed for a period of 90 days. The IRB (Comité de Protection des Personnes Sud-Est II) approved the study (ref. 2015-42-2) and this study was registered at clinicaltrials.gov (NCT 02638779).

The validation cohort is derived from the MIPrea (Marqueurs Immunitaires Pronostiques en Réanimation) study (8) that enrolled patients aged > 18 years old with an expected length of stay > 2 days and SIRS criteria (9) between December 2009 and June 2011 in six French ICUs (approval CIC IRB #5044). This validation cohort consisted exclusively in patients with sepsis. In this study, we also analyzed biological data at day 5-7.

### Intended management

Septic patients were treated according to the guidelines of the Surviving Sepsis Campaign guidelines (10). Patients received intravenous broad-spectrum antibiotics, depending on the presumed site of infection, previous antibiotic treatment and known colonization with antibiotic-resistant bacteria. Antimicrobial treatment was deescalated to narrower spectrum after identification of the responsible pathogen. Source control measures, such as surgery or removal of infected devices, were applied when necessary.

After initial stabilization using the "Parkland formula" for fluid resuscitation (11), burn patients were evaluated quickly to assess the need for debridement, escharotomy and fasciotomy in case of compartment syndrome. Opioids were used for pain control and sedation in addition to hypnotics. Patients underwent several surgical procedures for cleaning, non-viable tissue removal, skin graft and or amputation according to local aspect.

Initial management of severe trauma aimed to control the post-traumatic hemorrhage, including surgical procedures for damage-control and medical treatment of coagulopathy, and to stabilize hemodynamics with vasopressors if required. Patients were intubated and mechanically ventilated if alteration of consciousness, for preventing hypoxia. A restricted strategy of blood transfusion was applied and targeted hemoglobin of 70 to 90 g/L. Pain management was based on opioids administration, combined with regional anesthesia if adapted, or associated to hypnotics in sedated patients. Patients could undergo several surgical procedures according to location of trauma (12).

### Definitions

Severity at admission was assessed by the Simplified Acute Physiology Score 2 and the Sequential Organ Failure Assessment (SOFA) scores in septic patients and using the ISS score in trauma patients (13,14). ICU-acquired infections were defined as any new onset of probable or definite infection that developed after 48 h from ICU admission. Only the first episode of ICU-acquired infection was considered for analysis. ICU-acquired pneumonia was diagnosed according to the American Thoracic Society criteria (15). Patients with clinically suspected ventilator-associated pneumonia were usually subjected to a tracheobronchial aspirate with semiquantitative cultures. Diagnosis of catheter-related bloodstream infection required the growth of the same pathogen from both peripheral blood and catheter tip cultures, or from blood cultures sampled from the catheter and from venous puncture with a differential time to positivity > 120 min. Urinary tract infections, mostly catheter related, were diagnosed upon the association of systemic manifestations of infection and positive urine bacterial culture at ≥ 10⁵ CFU/mL. An independent adjudication committee composed of three clinicians not involved in study patients' recruitment or care reviewed the infections.

### Immune multiplex molecular tool

The multiplex molecular tool was performed as previously reported (9). Briefly, one PAXgene blood RNA tube (PreAnalytix, Hilden, Germany) was sampled at each time point, stabilized for at least 2h at room temperature after collection and frozen at -80°C following manufacturer's recommendations. mRNA expression was quantified using the FilmArray^{®} 2.0 instrument (bioMérieux) for automatic mRNA reverse transcription, amplification and further quantitative nested PCR of twenty-six genes *(ADGRE3, ARL14EP, BPGM, C3AR1, CCN81IP1, CD177, CD274, CD3D, CD74, CIITA, CTLA4, CX3CR1, GNLY, IFNgamma, IL10, IL1R2, IL1RN, IL7R, IP10*/*CXCL10, MDC1, OAS2, S100A9, TAP2, TDRD9, TNF* and *ZAP70).*

As a separate embodiment, the same protocol was used for automatic mRNA reverse transcription, amplification and further quantitative nested PCR of eleven genes (*C3AR1, CD177, CD3D, CD74, CIITA, CTLA4, CX3CR1, IFNG, IL1R2, S100A9* and *TAP2*). The results of this separate embodiment are also reported herein, after those based on the twenty-six genes reported above when relevant. When no information in this respect is indicated, the data set used is the data set obtained starting from the nested PCR of the twenty-six genes.

### Calculation of the transcriptomic score and statistical analysis

The normalized expression of individual transcripts of each gene evaluated by the multiplex molecular tool was first compared between healthy volunteers, patients with and without ICU-acquired infection. The receiver operator characteristics (ROC) curve for each gene to differentiate patient with and without ICU-acquired infection was then calculated. For genes with an area under the curve (AUC) > 0.70, we then calculated the optimal cut-point value using the Youden method as follows: sensitivity (%) + specificity (%) - 100. The cut-point value of each gene is then used to define a threshold value above or below which (depending on its increase or decrease in patients with ICU-acquired infection) each gene is allocated or not a point. The transcriptomic score was therefore the sum of all the values for a given patient.

### RESULTS

### Training cohort

In the REALISM cohort, 324 patients had a blood sampling at day 5-7. Among them 176 did not presented with an ICU-acquired pneumonia before day 5-7 blood sampling and were still in the ICU at that time point. Most patients included in this training set were admitted for trauma (n=72, 41%) and sepsis (n=68, 39%). The overall ICU mortality was 8%.

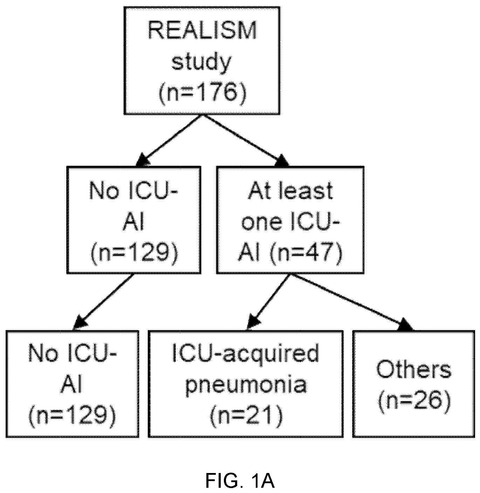

Overall, 47 patients presented at least one episode of ICU-acquired infection while 129 patients did not. Most ICU-acquired infections were ICU-acquired pneumonia (n=21, 37%) **(****figure 1A****).** Patients who presented an ICU-acquired infection had more frequent diabetes mellitus (21% vs. 14%) and chronic pulmonary disease (19% vs. 9%) when compared to patients without ICU-acquired infections **(Table 1).** The median duration of mechanical ventilation was longer in patients with ICU-acquired infection when compared to patients without (ICU-AI 15 days (95%IQR: 1 - 29) vs. 1 day (0 - 3), respectively).

**Table 1: Clinical characteristics of the discovery cohort**

| | | | **No ICU-AI (n=129)** | **ICU-AI (n=47)** |
|---|---|---|---|---|
| **Baseline characteristics** | | | | |
| | **Subgroup** | | | |
| | | **Burn** | 2 (2) | 14 (30) |
| | | **Sepsis/Septic shock** | 57 (44) | 11 (23) |
| | | **Surgery** | 11 (9) | 9 (19) |
| | | **Trauma** | 59 (46) | 13 (28) |
| | **Female qender** | | 33 (26) | 14 (30) |
| | **Aqe, years** | | 61 (47 - 73) | 59 (46 - 70) |

| | **Comorbidities** | | | |
|---|---|---|---|---|
| | | **Diabetes mellitus** | 19 (14) | 10 (21) |
| | | **Chronic renal failure** | 13 (9) | 1 (2) |
| | | **Chronic pulmonary disease** | 13 (9) | 9 (19) |
| | | **HIV infection** | 0 (0) | 0 (0) |
| | | **Solid tumors** | 22 (16) | 9 (19) |
| | | **Hematoloqical malignancy** | 0 (0) | 0 (0) |
| | **Body mass index, kg/m²** | | 25 (22 - 28) | 25 (22 - 29) |
| | | | | |

| **Parameters at ICU admission** | | | | |
|---|---|---|---|---|
| | **SOFA score** | | 6 (2 - 9) | 7 (5 - 9) |
| | **Lactate, mmol/L** | | 0,8 (0 - 2,6) | 1,2 (0 - 2,6) |
| | **Mechanical ventilation** | | 69 (53) | 37 (79) |
| | **Norepinephrine or epinephrine use** | | 88 (68) | 40 (85) |
| | | | | |

| **Outcomes** | | | | |
|---|---|---|---|---|
| | **Duration of mechanical ventilation, days** | | 1 (0 - 3) | 15 (1 - 29) |
| | **Death in ICU** | | 7 (5) | 8 (17) |

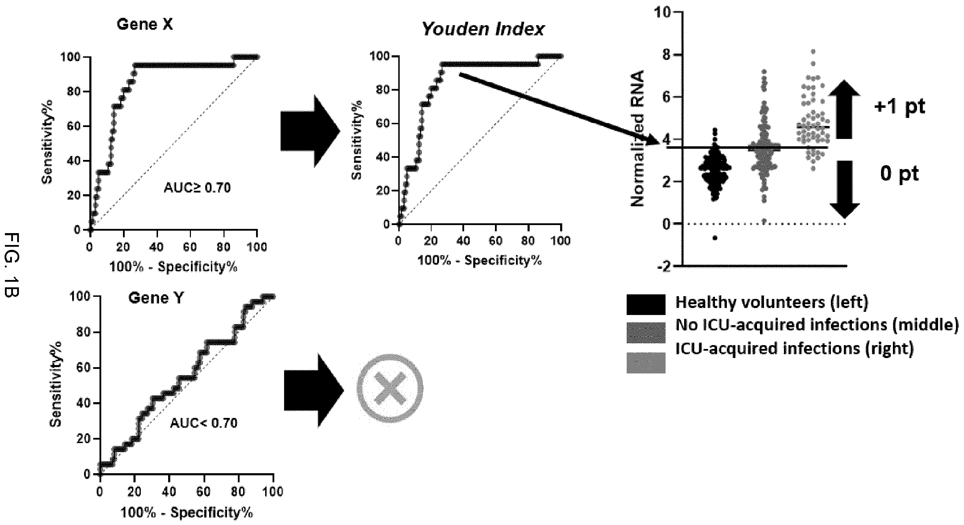

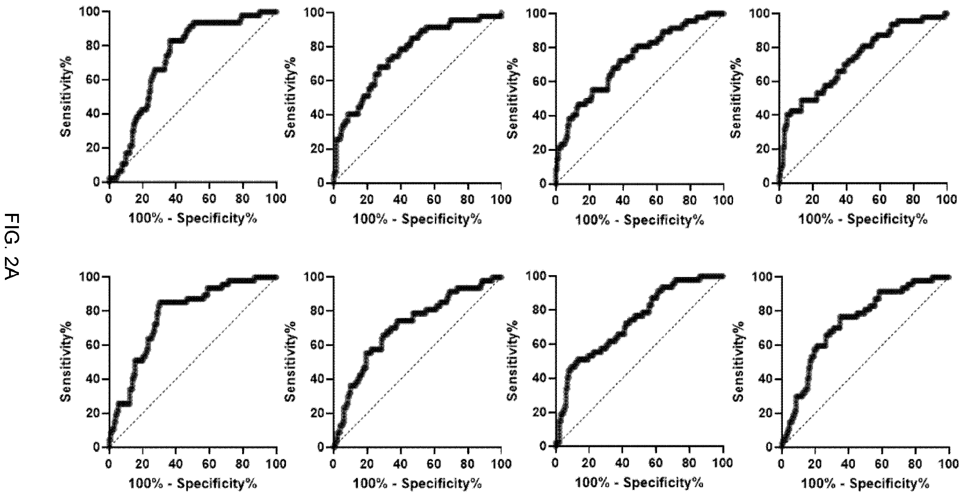

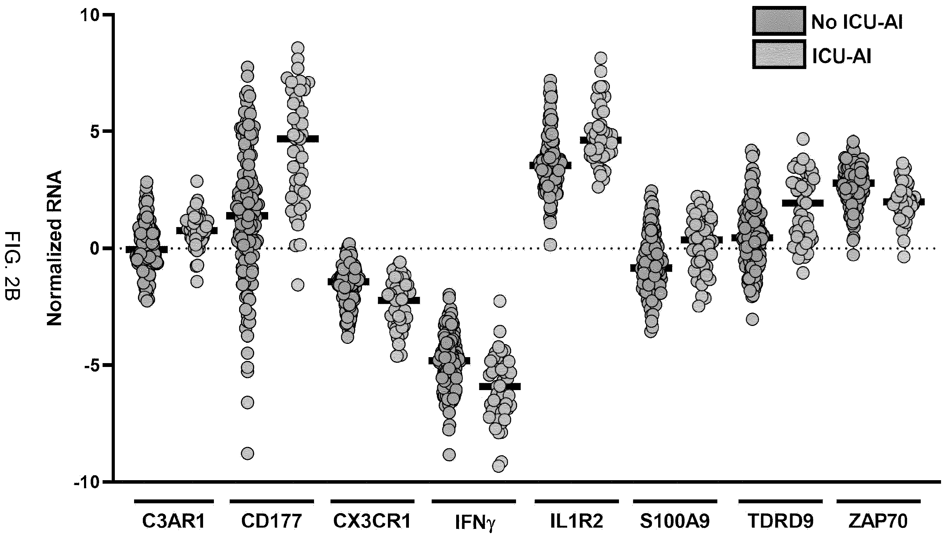

Using our approach **(****Figure 1B****),** among the 26 genes included in the multiplex molecular tool, 8 transcripts had an AUC value upper than 0.70 to classify patients with or without ICU-acquired infection, namely *C3AR1, CD177, CX3CR1, IFNγ*, *IL1R2, S100A9, TDRD9* and *ZAP70* **(****figure 2A****).** Normalized mRNA transcript expression was increased in patients with ICU-acquired infections except for *CX3CR1, IFNγ* and *ZAP70* **(****figure 2B****).** The main diagnosis performances and cut-point value for each of the 8 transcripts are summarized in **Table 2.**

When, according to another embodiment, the 11 genes included in the multiplex molecular tool have been used, it could be determined that 6 transcripts had an AUC value upper than 0.75 to classify patients with or without ICU-acquired infection, namely *C3AR1, CD177, CX3CR1, IFNγ*, *IL1R2* and *S100A9* **(data not shown).** Normalized mRNA transcript expression was increased in patients with ICU-acquired infections except for *CX3CR1* and *IFNγ* **(data not shown).**

**Table 2: Table summarizing diagnostic performances of identified genes in the discovery cohort.**

| **Genes** | **AUC** | **Cut-off value** | **Specificity** | **Sensibility** |
|---|---|---|---|---|
| **C3AR1** | 0,73 (0.65 - 0.80) | > 0,360 | 0,63 (0,55 - 0,71) | 0,83 (0,70 - 0,91) |
| **CD177** | 0,76 (0,68 - 0,84) | > 2,867 | 0,73 (0,65 - 0,80) | 0,68 (0,54 - 0,79) |
| **CX3CR1** | 0,73 (0,64 - 0,81) | < -1,679 | 0,62 (0,53 - 0,70) | 0,72 (0,59 - 0,83) |
| **IFNγ** | 0,73 (0,64 - 0,81) | < -6,601 | 0,95 (0,90 - 0,98) | 0,40 (0,27 - 0,55) |
| **IL1R2** | 0,77 (0,70 - 0,84) | > 3,894 | 0,70 (0,61 - 0,77) | 0,85 ( 0,72 - 0,93) |
| **S100A9** | 0,71 (0,62 - 0,79) | > -0,189 | 0,70 (0,62 - 0,77) | 0,66 (0,52 - 0,78) |
| **TDRD9** | 0,74 (0,65 - 0,82) | > 1,923 | 0,87 (0,80 - 0,92) | 0,51 (0,37 - 0,64) |
| **ZAP70** | 0,74 (0,65 - 0,81) | < 2,482 | 0,65 (0,57 - 0,73) | 0,71 (0,50 - 0,86) |

We built the transcriptomic score (TScore) using the threshold values obtained with the Youden technique on the ROCs for each transcript. For each gene analyzed individually, patients with a value above the threshold (for *C3AR1, CD177, IL1R2, S100A9 and TDRD9*) or below threshold (for *CX3CR1, IFNγ* and *ZAP70*) were assigned a point. Conversely, genes that do not meet these criteria were assigned a value of 0. The TScore, which varies between 0 and 8, is the sum of the values for each of the 8 genes **(****figure 3A****).** The AUC value of this TScore performed at day 5-7 is 0.86 ((0.80 - 0.92) in distinguishing patients with or without at least one episode of ICU-acquired infection during their ICU stay **(****figure 3B****).** Using the Youden index, the optimal value of the score is 2. We therefore defined a high-risk population of patients with a TScore upper to 2 and a low-risk population of patients with a TScore lower or equal to 2.

When applied to the whole population of the training set, patients with a TScore between 3 and 8 (n=87, 49%) presented a higher proportion of ICU-acquired infection when compared to patients with a TScore lower or equal to 3 (49% versus 4%, respectively) **(****figure 3C****).** In these high-risk patients, ICU-acquired infections were more frequently pneumonia (n=19, 49%) when compared to low-risk patients (n=2, 11%) **(****figure 3D****).** Furthermore, they experienced a longer median ICU length of stay (13 days, 25^{th}-75^{th} IQR: 8 - 30 versus 7 days, 25^{th}-75^{th} IQR: 6-9, p<0.001) when compared to patients in the low-risk TScore group **(****figure 3E****).** The ICU mortality in the high-risk TScore group was higher than the mortality of the patients in the low-risk TScore group (15% versus 2%, p<0.001) **(****Figure 3F****).** All parameters are summarized in **Table 3.**

We also built the transcriptomic score TScore of the 6 transcripts selected from the 11 genes included in the multiplex molecular tool according to the other embodiment reported herein, using the threshold values obtained with the Youden technique on the ROCs for each transcript. For each gene analyzed individually, patients with a value above the threshold (for *C3AR1, CD177, IL1R2* and *S100A9*) or below threshold (for *CX3CR1* and *IFNγ*) were assigned a point. Conversely, genes that do not meet these criteria were assigned a value of 0. This TScore therefore varies between 0 and 6, and is the sum of the values for each of the 6 genes **(data not shown).** The AUC value of this TScore is 0.84 (0.77 - 0.89) in distinguishing patients with or without at least one episode of ICU-acquired infection at day 5-7 **(data not shown).** In a very interesting way, it has been previously disclosed that the combination of the 6 transcripts *C3AR1, CD177, IL1R2, S100A9, CX3CR1* and *IFNγ* exhibited a performance in terms of AUC value of about 0.79 for assessing the risk of ICU-acquired infection (see WO2022/008828, page 156). Therefore, applying the TScore method using the same 6 transcripts allows to better distinguish patients with or without at least one episode of ICU-acquired infection.

Using the Youden index, the optimal value of the score is 2. We therefore defined a high-risk population of patients with a TScore upper to 2 and a low-risk population of patients with a TScore lower or equal to 2. When applied to the whole population of the training set, patients with a TScore between 3 and 6 (n=82, 46%) presented a higher proportion of ICU-acquired infection when compared to patients with a TScore lower or equal to 2 (59% versus 9%, respectively) **(data not shown).** In these high-risk patients, ICU-acquired infections were more frequently pneumonia (n=20, 49%) when compared to low-risk patients (n=1, 11%) **(data not shown).** Furthermore, they experienced a longer median ICU length of stay (15 days, 25^{th}-75^{th} IQR: 9 - 31 versus 7 days, 25^{th}-75^{th} IQR: 6-9, p<0.001) when compared to patients in the low-risk TScore group **(data not shown).** Therefore, in this configuration also, the ICU mortality in the high-risk TScore group was higher than the mortality of the patients in the low-risk TScore group (16% versus 2%, p<0.001) **(data not shown).**

**Table 3: table summarizing the clinical characteristics and outcomes of patients with a Tscore between 0 and 2 (low risk) or upper or equal to 3 (high risk) (SOFA: Sequential organ failure assessment score).**

| | | | **Tscore [0-2] (n=89)** | **Tscore [3-8] (n=87)** |
|---|---|---|---|---|
| **Baseline characteristics** | | | | |
| | **Subgroup** | | | |
| | | **Burn** | **0 (0)** | **16 (18)** |
| | | **Sepsis/Septic shock** | **29 (33)** | **39 (45)** |
| | | **Surgery** | **5 (6)** | **15 (17)** |
| | | **Trauma** | **55 (62)** | **17 (20)** |
| | **Female gender** | | **20 (22)** | **27 (31)** |
| | **Age, years** | | **54 (43 - 66)** | **65 (49 - 76)** |
| | **Body mass index, kg/m²** | | **24.7 (22.9 - 27.4)** | **26 (22.7 - 29.6)** |
| | | | | |

| **Parameters at ICU admission** | | | | |
|---|---|---|---|---|
| | **SOFA score** | | **5 (1 - 8)** | **8 (5 - 11)** |
| | **Lactate, mmol/L** | | **0 (0 - 2.1)** | **1.6 (0 - 3.2)** |
| | **Mechanical ventilation** | | **41 (46)** | **65 (75)** |
| | **Norepinephrine use** | | **54 (61)** | **74 (85)** |
| | | | | |

| **Outcomes** | | | | |
|---|---|---|---|---|
| | **ICU length of stay, days** | | **7 (6 - 9)** | **13 (8 - 30)** |
| | **ICU-acquired infections** | | **4 (4)** | **43 (49)** |
| | | **Pneumonia** | **2** | **19** |
| | | **Urinary tract infection** | **2** | **9** |
| | | **Others** | **0** | **15** |
| | **Death in the ICU** | | **2 (2)** | **13 (15)** |

In the training set, a Wilcoxon-Mann-Whitney test was performed to determine potential relevant clinical and biological variables of interest for predicting the occurrence of ICU-acquired infections. Of the variables analyzed, only SOFA score at day 6 and the TScore showed a statistically different distribution between ICU-acquired infections and no ICU-acquired infections. In a multivariate model, the TScore remained independently associated with ICU-acquired infections occurrence **(Table 4).** A type I ANOVA was applied, varying the order of introduction of the explanatory variables in the model, to determine the own contribution of the explanatory variables TScore and SOFA at day 6 to the minimization of deviance, as well as their joint contribution. The model thus created explains 25.4% of the total deviance, with 81% being explained by the TScore alone, 16.8% of the deviance explained by the TScore and the SOFA at day 6 together, and 1.7% by SOFA at day 6 alone. TScore therefore only slightly overlaps with information already explained by the main clinical variables in the REALISM database and the addition of such variables to TScore seems of little relevance in regard of the low proportion of deviance they explained in the model.

**Table 4: univariate analysis of relevant clinical parameters between low-risk and high-risk patients in the discovery cohort followed by the multivariate analysis including parameters statistically significant in univariate analysis**

| **Univariate analysis** | **p-value** | |
|---|---|---|
| **Age, years** | **0.89** | |
| **Charlson score** | **0.66** | |
| **SAPSII score** | **0.46** | |
| **SOFA at day 6** | **0.0079** | |
| **Tscore** | **3.8.10⁻⁹** | |
| | | |

| **Multivariate analysis** | **Estimate** | **p-value** |
|---|---|---|
| **SOFA at day 6** | **-0,06** | **0.32** |
| **Tscore** | **0.6** | **6.4.10⁻⁹** |

Finally, we investigated the expression level of several biomarkers previously reported to be associated with poor outcomes in ICU patients (16). Interestingly, we found that the monocytic HLA-DR level of expression was decreased in high-risk patients. Similarly, the concentration of the anti-inflammatory cytokine IL10 was increased in high-risk patients. Only immature neutrophils proportion was similar in both groups **(****Figure 4****).** Finally, when we apply our model to the training cohort according to the initial aggression (sepsis, burns, trauma and surgery), we find the same capabilities of the TScore to identify a group at high risk of complications **(Table 5).**

**Table 5: clinical parameters and outcomes of patients with a Tscore between 0 and 2 (low risk) or upper or equal to 3 (high risk) sub-classified according to their subgroup**

| | **Global population** | **Burn** | | **Sepsis** | | **Surgery** | | **Trauma** | |
|---|---|---|---|---|---|---|---|---|---|
| | **176 patients** | **Low Risk (n=0)** | **High Risk (n=16)** | **Low Risk (n=29)** | **High Risk (n=39)** | **Low Risk (n=5)** | **High Risk (n=15)** | **Low Risk (n=55)** | **High Risk (n=17)** |
| **SAPSII at admission** | **36 [25-47]** | **/** | **37 [28-42]** | **40 [36-49]** | **53 [45-62]** | **26 [25-27]** | **25 [22-31]** | **26 [21-37]** | **35 [29-45]** |
| **SOFA at admission** | **7 [3-9]** | **/** | **6 [4-8]** | **8 [6-10]** | **10 [8-12]** | **3 [3-4]** | **5 [2-6]** | **2 [0-7]** | **7 [3-11]** |
| **Lactate at admission** | **0.8 [0.0-2.6]** | **/** | **1.8 [0.0-3.6]** | **1.3 [0.0-2.1]** | **2.5 [0.0-3.6]** | **0.0 [0.0-0.0]** | **0.0 [0.0-0.0]** | **0.0 [0.0-2.1]** | **1.6 [0.0-2.8]** |
| **Mechanical ventilation** | **106 (60)** | **/** | **14 (87)** | **20 (69)** | **34 (87)** | **0 (0)** | **3 (20)** | **21 (38)** | **14 (82)** |
| **Norepinephrine use** | **128 (72)** | **/** | **14 (8)** | **27 (93)** | **38 (97)** | **2 (40)** | **9 (60)** | **25 (45)** | **13 (76)** |
| **ICU LOS** | **9 [7-15]** | **/** | **53 [39-62]** | **7 [6-8]** | **13 [9-19]** | **7 [6-7]** | **8.0 [6.5-9.0]** | **8 [6-9]** | **18 [12-33]** |
| **MV duration** | **1 [0-6]** | **/** | **26 [16-44]** | **3 [0-5]** | **3 [1-8]** | **0.0 [0.0-0.0]** | **0.0 [0.0-0.0]** | **0 [0-1]** | **5 [1-23]** |
| **ICU death** | **15 (8)** | **/** | **4 (25)** | **2 (7)** | **7 (18)** | **0 (0)** | **0 (0)** | **0 (0)** | **2 (12)** |
| **ICU-AI** | **47 (27)** | **/** | **14 (87)** | **0 (0)** | **11 (28)** | **0 (0)** | **6 (40)** | **4 (7)** | **9 (53)** |

### Validation cohort

For the validation cohort, we assessed the diagnosis performance of the TScore in septic patients from the MIPrea cohort. The proportion of high-risk patients with a TScore between 3 and 8 was higher in the MIPrea cohort when compared to the REALISM cochort (62% versus 49%, respectively). High-risk patients were most severe at ICU admission, with a higher SOFA score (10, 25^{th}-75^{th} IQR: 7 - 13 versus 8, 25^{th}-75^{th} IQR: 5 -11) and higher rate of mechanical ventilation (87% versus 75%) **(Table 6).** Conversely, the proportion of patients with a TScore between 3 and 6 was also higher in the MIPrea cohort when compared to the REALISM cochort (56% versus 46%, respectively). High-risk patients were also most severe at ICU admission, with a higher SOFA score (11, 25^{th}-75^{th} IQR: 8 - 13 versus 8, 25^{th}-75^{th} IQR: 6 - 10) and higher rate of mechanical ventilation (89% versus 77%) **(data not shown).**

**Table 6: table summarizing the clinical characteristics and outcomes of patients with a Tscore between 0 and 2 (low risk) or upper or equal to 3 (high risk) (SOFA: Sequential organ failure assessment score)**

| | | | **T score [0-2] (n=97)** | **Tscore [3-8] (n=160)** |
|---|---|---|---|---|
| **Baseline characteristics** | | | | |
| | **Female gender** | | **34 (35)** | **63 (39)** |
| | **Age, years** | | **64 (54 - 75)** | **66 (57 - 77)** |
| | **Comorbidities** | | | |
| | | **Diabetes mellitus** | **22 (22)** | **25 (15)** |
| | | **Chronic renal failure** | **10 (10)** | **11 (7)** |
| | | **Chronic pulmonary disease** | **32 (33)** | **44 (27)** |
| | | **HIV infection** | **1 (1)** | **1 (1)** |
| | | **Solid tumors** | **19 (19)** | **44 (28)** |
| | | **Hematoloqical malignancy** | **5 (5)** | **1 (1)** |

| **Parameters at ICU admission** | | | | |
|---|---|---|---|---|
| | **SAPS II score** | | **56 (40 - 69)** | **60 (48 - 70)** |
| | **SOFA score** | | **8 (6 - 10)** | **10 (7 - 13)** |
| | **Lactate, mmol/L** | | **1.8 (1.2 - 2.9)** | **2.4 (1.6 - 3.6)** |
| | **Mechanical ventilation** | | **75 (77)** | **140 (87)** |
| | **Norepinephrine or epinephrine use** | | **62 (64)** | **123 (77)** |
| | | | | |

| **Outcomes** | | | | |
|---|---|---|---|---|
| | **ICU length of stay, days** | | **9 (7 - 14)** | **12 (8 - 20)** |
| | **Duration of mechanical ventilation, days** | | **6 (3 - 14)** | **9 (5 - 16)** |
| | **ICU-acquired infections** | | **18 (18)** | **47 (30)** |
| | | **Pneumonia** | **10** | **32** |
| | | **Urinary tract infection** | **6** | **8** |
| | | **Others** | **2** | **7** |
| | **Death in ICU** | | **13 (13)** | **47 (29)** |

ICU-acquired infections were more frequent in high-risk patients (30% versus 18%, p=0.06) **(****Figure 5A****),** with a lower proportion of urinary tract infections **(****Figure 5B****).** The median ICU length of stay was longer in high-risk patients (12 days, 25^{th}-75^{th} IQR: 8 - 20 versus 9 days, 25^{th}-75^{th} IQR: 7 -14, p=0.003) **(****Figure 5C****).** ICU mortality was also higher in high-risk patients (20% versus 13%, p=0.006) **(****Figure 5D****).**

### DISCUSSION

Two groups of patients with different risks of poor outcome, including ICU-acquired infections, mortality and length of ICU stay were identified using a transcriptomic score based on the expression level of 6 or 8 genes. Relative to low-risk patients, patients in the high-risk group had an increased risk of ICU-acquired infections, even after adjustment for SOFA score, longer duration of ICU stay and higher mortality. High-risk assignment at day 5-7 was associated with lower mHLA-DR values and higher circulating IL10 values.

Owing to early identification of sepsis, rapid initiation of antibiotics and organ support therapies, many septic patients now survive the early phase of sepsis. However, they are therefore exposed to a higher risk of secondary infections that are associated with several markers of immune dysfunction such as downregulated monocytic HLA-DR expression (17). Despite a robust pathophysiological rationale, several randomized clinical trials have failed in showing a beneficial effect of immune modulating therapies over the last decades (18). One of the hypothesis for this failure rely on the important heterogeneity in septic patients and suggest to stratify patients on the basis of immune status in order to identify those who are more likely to benefit from these therapies 19). Such stratification requires reliable, relevant, rapid and decision-making monitoring tools.

With this aim, assessment of the global differential gene expression within blood has provided encouraging results in the early phase of sepsis (20). However, whether most of the studies fostered on mortality (21, 22), only a few genes were reported as relevant biomarkers to predict ICU-acquired infections (23). Indeed, it is difficult to identify genes associated with a higher risk of ICU-acquired infection and several hypotheses can be formulated to explain it. First, blood cellular composition and gene expression vary rapidly over time depending on patient-related and management-related parameters. Second, the onset of sepsis remains unknown in most cases. Therefore, the simultaneous assessment of the expression of several genes appears as an interesting approach in order to pick up a signal whatever the time when the sample is performed (9). To do so, we took advantages of the advent of multiplex molecular platforms such as the bioMérieux FilmArray^{®} System that allows the realization of rapid and reliable evaluation of the expressions of several genes. We performed our analysis between 5 and 7 days after ICU admission, a timeframe which we believe may shift away from abnormalities potentially related to the initial heterogeneity of sepsis, but the analysis could have also been performed at another time period following ICU admission. Furthermore, transcriptomic results were unaffected by the occurrence of ICU-acquired infections that were absent at this timepoint.

Over the last years, machine learning (ML) algorithms have gained increasing interest for classification, unsupervised clustering or dimensionality reduction tasks of large data sets. ML tools use data-driven algorithms and statistical models to analyze data sets and then draw inferences from identified patterns or make predictions based on them. However, ML algorithms based on gene-expression performances in predicting ICU-acquired infections remains similar to usual severity scores (24). These poor results could be related to the mismatch between two main assumptions of the ML algorithms and the characteristics of the clinical and biological data from which the algorithms are derived. First, ML algorithms assume the absence of covariate shift between training and test set. This assumption is frequently violated in ICU given the important heterogeneity in patient's characteristics. Second, ML models intrinsically generates different features importance among dependent variables in order to fit as well as possible to the data. This assumption may be responsible for data overfitting and therefore limit generalization of the models.

Based on these observations, we built the TScore considering that 1/ each gene is independently associated with ICU-acquired infection and has an equal importance in the score without any weighting and 2/ that genes included in the score are mutually independent. This possibly allows for the capture of different pathophysiological mechanisms that would reflect an alteration of the immune system. This simplistic approach does not take into account the possible interactions between genes in signaling pathways that may be involved. However, given the differences among studies regarding the setting and the heterogeneity in septic patients, we believe this approach is appropriate in capturing relevant information in heterogeneous patients.

We found that the TScore was associated with the occurrence of ICU-acquired infections independently of the clinical variables usually associated with it. Furthermore, we found in high-risk patients biological features of immune suppression, with low levels of monocytic HLA-DR and high levels of the anti-inflammatory cytokine IL-10. These data suggest that the TScore is able to identify patients at risk of ICU-acquired infections based on circulating immune alterations. Therefore, our results suggest that this TScore could be used as a stratifying tool to identify patients likely to benefit from immune therapies.

This score was build using a cohort of critically ill patients admitted in the ICU with various etiologies, including severe infections, burns, trauma and surgery. In line with previously published results, we postulated that immune alterations in circulating cells are shared among various medical conditions (10). We then were able to validate this approach on a cohort of patients exhibiting exclusively a severe infection, which shows the robustness of the score.

This study acknowledges several limitations. First, we provided validation in an historical independent cohort and not in a prospectively collected cohort. Second, even if the results were repeated on a validation cohort, our results need to be evaluated in a large cohort and performed in a prospective way to confirm the performances of this test. Third, our results are based on whole-blood leukocyte populations that may differ among patients. Therefore, we cannot exclude that our results rather reflect differences in leukocytes populations rather than intracellular gene-expression differences. Fourth, the PCR multiplex was performed at day 5-7 after ICU admission. This limits the diagnostic performance of this test to the time period to which it is performed. The TScore must be validated and/or adapted at several time points, which should be possible thanks to the agility and plasticity of the platform.

### CONCLUSION

Using a multiplex molecular platform, we built a transcriptomic score based on simultaneous assessment of the expression of six genes at day 5-7 after ICU admission that identifies a subgroup of patients at high-risk to develop ICU-acquired infections. The host response that is detected in high-risk patients is associated with known biomarkers of immune dysfunction and provide a useful and reliable companion diagnostic tool to further develop immune modulating drugs evaluation in sepsis.

The transcriptomic score provides a useful and reliable companion diagnostic tool to further develop immune modulating drugs in sepsis in the context of personalized medicine.

### BIBLIOGRAPHY

1. Singer M, Deutschman CS, Seymour CW, Shankar-Hari M, Annane D, Bauer M, et al. The Third International Consensus Definitions for Sepsis and Septic Shock (Sepsis-3). JAMA. 23 févr 2016;315(8):801.
2. van Vught LA, Klein Klouwenberg PMC, Spitoni C, Scicluna BP, Wiewel MA, Horn J, et al. Incidence, Risk Factors, and Attributable Mortality of Secondary Infections in the Intensive Care Unit After Admission for Sepsis. JAMA. 12 avr 2016;315(14):1469-79.
3. Llitjos JF, Bounab Y, Rousseau C, Dixneuf S, Rimbault B, Chiche JD, et al. Assessing the Functional Heterogeneity of Monocytes in Human Septic Shock: a Proof-of-Concept Microfluidic Assay of TNFα Secretion. Front Immunol. 2021;12:686111.
4. Llitjos JF, Gassama A, Charpentier J, Lambert J, de Roquetaillade C, Cariou A, et al. Pulmonary infections prime the development of subsequent ICU-acquired pneumonia in septic shock. Ann Intensive Care. 15 mars 2019;9(1):39.
5. Llitjos JF, Bredin S, Lascarrou JB, Soumagne T, Cojocaru M, Leclerc M, et al. increased susceptibility to intensive care unit-acquired pneumonia in severe COVID-19 patients: a multicentre retrospective cohort study. Ann Intensive Care. 29 janv 2021;11(1):20.
6. Tawfik DM, Vachot L, Bocquet A, Venet F, Rimmelé T, Monneret G, et al. immune Profiling Panel: A Proof-of-Concept Study of a New Multiplex Molecular Tool to Assess the immune Status of Critically III Patients. J Infect Dis. 21 juill 2020;222(Supplement_2):S84-95.
7. Venet F, Textoris J, Blein S, Rol ML, Bodinier M, Canard B, et al. immune Profiling Demonstrates a Common immune Signature of Delayed Acquired Immunodeficiency in Patients With Various Etiologies of Severe Injury. Crit Care Med. 16 sept 2021;
8. Friggeri A, Cazalis MA, Pachot A, Cour M, Argaud L, Allaouchiche B, et al. Decreased CX3CR1 messenger RNA expression is an independent molecular biomarker of early and late mortality in critically ill patients. Crit Care Lond Engl. 30 juin 2016;20(1):204.
9. Bone RC, Balk RA, Cerra FB, Dellinger RP, Fein AM, Knaus WA, et al. Definitions for Sepsis and Organ Failure and Guidelines for the Use of Innovative Therapies in Sepsis. Chest, juin 1992;101(6):1644-55.
10. Rhodes A, Evans LE, Alhazzani W, Levy MM, Antonelli M, Ferrer R, et al. Surviving Sepsis Campaign: International Guidelines for Management of Sepsis and Septic Shock: 2016. Crit Care Med. mars 2017;45(3):486-552.
11. Baxter CR. Fluid volume and electrolyte changes of the early postburn period. Clin Plast Surg. oct 1974;1(4):693-703.
12. Spahn DR, Bouillon B, Cerny V, Duranteau J, Filipescu D, Hunt BJ, et al. The European guideline on management of major bleeding and coagulopathy following trauma: fifth edition. Crit Care. déc 2019;23(1):98.
13. Le Gall JR. A New Simplified Acute Physiology Score (SAPS II) Based on a European/North American Multicenter Study. JAMA J Am Med Assoc. 22 déc 1993;270(24):2957.
14. Vincent JL, de Mendonça A, Cantraine F, Moreno R, Takala J, Suter PM, et al. Use of the SOFA score to assess the incidence of organ dysfunction/failure in intensive care units: results of a multicenter, prospective study. Working group on « sepsis-related problems » of the European Society of Intensive Care Medicine. Crit Care Med. nov 1998;26(11): 1793-800.
15. Kalil AC, Metersky ML, Klompas M, Muscedere J, Sweeney DA, Palmer LB, et al. Management of Adults With Hospital-acquired and Ventilator-associated Pneumonia: 2016 Clinical Practice Guidelines by the Infectious Diseases Society of America and the American Thoracic Society Clin Infect Dis. 1 sept 2016;63(5):e61-111.
16. Tremblay JA, Peron F, Kreitmann L, Textoris J, Brengel-Pesce K, Lukaszewicz AC, et al. A stratification strategy to predict secondary infection in critical illness-induced immune dysfunction: the REALIST score. Ann Intensive Care. 17 août 2022;12(1):76.
17. Landelle C, Lepape A, Voirin N, Tognet E, Venet F, Bohé J, et al. Low monocyte human leukocyte antigen-DR is independently associated with nosocomial infections after septic shock. Intensive Care Med. nov 2010;36(11):1859-66.
18. Cavaillon J, Singer M, Skirecki T. Sepsis therapies: learning from 30 years of failure of translational research to propose new leads. EMBO Mol Med [Internet]. 7 avr 2020 [cité 7 juin 2022];12(4). Disponible sur: https://onlinelibrary.wiley.com/doi/10. 15252/emmm.201810128
19. van de Veerdonk FL, Giamarellos-Bourboulis E, Pickkers P, Derde L, Leavis H, van Crevel R, et al. A guide to immunotherapy for COVID-19. Nat Med. janv 2022;28(1):39-50.
20. Davenport EE, Burnham KL, Radhakrishnan J, Humburg P, Hutton P, Mills TC, et al. Genomic landscape of the individual host response and outcomes in sepsis: a prospective cohort study. Lancet Respir Med. avr 2016;4(4):259-71.
21. Sweeney TE, Perumal TM, Henao R, Nichols M, Howrylak JA, Choi AM, et al. A community approach to mortality prediction in sepsis via gene expression analysis. Nat Commun. déc 2018;9(1):694.
22. Scicluna BP, van Vught LA, Zwinderman AH, Wiewel MA, Davenport EE, Burnham KL, et al. Classification of patients with sepsis according to blood genomic endotype: a prospective cohort study. Lancet Respir Med. oct 2017;5(10):816-26.
23. Peronnet E, Venet F, Maucort-Boulch D, Friggeri A, Cour M, Argaud L, et al. Association between mRNA expression of CD74 and IL10 and risk of ICU-acquired infections: a multicenter cohort study. Intensive Care Med. juill 2017;43(7):1013-20.
24. Brakenridge SC, Starostik P, Ghita G, Midic U, Darden D, Fenner B, et al. A Transcriptomic Severity Metric That Predicts Clinical Outcomes in Critically III Surgical Sepsis Patients Crit Care Explor. 14 oct 2021;3(10):e0554

## Claims

1. A method of determining, from an assayed sample or a population of assayed samples previously drawn from patients, a transcriptomic score (TScore) suitable for assessing a risk of adverse clinical outcome for patients in a clinical setting, said method comprising:
a. Measuring gene expression values on a patient sample or a population of samples previously obtained from patients, or if the method is computer-implemented retrieving such gene expression values, for at least two distinct genes selected among a predetermined set of genes,and
b. For each gene of the predetermined set of genes, and for each sample, comparing the measurements obtained or retrieved in step a. to predetermined threshold values identifying the assayed sample as pertaining to a patient presenting an infection or not presenting an infection, in particular an ICU-acquired infection, and/or determining on the basis of the measurements obtained or retrieved in step a., a receiver operator characteristics (ROC) curve aimed at representing the diagnostic ability of the gene expression value measured for said gene to identify the assayed sample as pertaining to a patient presenting an infection or not presenting an infection, in particular an ICU-acquired infection, and
c. For the assayed sample or each patient sample of the population of assayed samples, calculating a transcriptomic score where:
i. For each gene of the predetermined set of genes analyzed individually, if the measured gene expression value matches the condition by which the sample is to be classified as a sample pertaining to a patient presenting an infection, in particular an ICU-acquired infection, assigning one point to the gene, and
ii. Summing up the number of points obtained for all the genes of the predetermined set of genes, thereby obtaining a transcriptomic score for the assayed sample, and
iii. Optionally, providing the transcriptomic score as an output value.

2. The method of claim 1, which is carried out on a population of assayed samples previously drawn from patients for which it is known for a part of the samples whether they have been drawn from patients that have presented at least one episode of infection since their admission in a care unit, notably their admission in an ICU, which further comprises:
a. Prior to step c., a step of further selecting, within the predetermined set of genes of a., genes for which the AUC value of the ROC curves of step b. are at least 0.70, and optionally providing the updated predetermined set of genes as an output value, and/or
b. Prior to step c., a step of determining a threshold value for each gene of the predetermined set of genes that is analyzed, in particular through calculation of the Youden Index on the ROC curve determined in step b., more particularly wherein the threshold value is the value for which the Youden Index is maximized, and optionally providing the threshold value for the gene as an output value.

3. The method of claim 1 or claim 2, wherein a gene expression value is measured through mRNA expression levels, in particular through a multiplex assay involving the plurality of assayed genes, or by sequencing.

4. The method of any one of claims 1 to 3, wherein a gene expression value is measured by RT-PCR, in particular RT-qPCR, more particularly nested RT-qPCR, especially a multiplex RT-PCR or RT-qPCR or nested RT-qPCR.

5. The method of any one of claims 1 to 4, wherein:
a. the genes of the predetermined set of genes of step a. of claim 1 are selected among: *ADGRE3, ARL14EP, BPGM, C3AR1, CCN81IP1, CD177, CD274, CD3D, CD74, CIITA, CTLA4, CX3CR1, GNLY, IFNgamma, IL10, IL1R2, IL1RN, IL7R* , *IP10*/*CXCL10, MDC1, OAS2, S100A9, TAP2, TDRD9, TNF* and *ZAP70,* and/or
b. the predetermined set of genes of step a. of claim 1 or of step a. of claim 2 encompasses at least 4 genes, preferably at least 6 or 8 genes, in particular encompasses 6 or 8 genes, and/or
c. the predetermined set of genes of step a. or of step a. of claim 2 consists in the following genes: C3AR1, CD177, CX3CR1, IFNgamma, IL1R2, S100A9, TDRD9 and ZAP70 or consists in the following genes: C3AR1, CD177, CX3CR1, IFNgamma, IL1R2 and S100A9.

6. The method of any one of claims 1 to 5, wherein the assayed biological sample(s) is (are) drawn from patient(s) which have been admitted in a reanimation unit, an intensive or continuing care unit, and/or susceptible to be suffering of, or susceptible of being diagnosed with, or suffering from sepsis, preferably is (are) drawn from patient(s) which have been admitted in an intensive care unit (ICU), and more preferably between days 5 and 7 of the ICU stay.

7. The method of any one of claims 1 to 6, wherein gene expressions values are measured through mRNA expression levels and the genes of the predetermined set of genes are selected among: C3AR1, CD177, CX3CR1, IFNgamma, IL1R2, S100A9, TDRD9 and ZAP70, wherein the predetermined threshold value of step b. of claim 1 are, for each gene respectively, as follows:
- C3AR1: 0.360
- CD177: 2.867
- CX3CR1: -1.679
- IFNgamma: -6.601
- IL1R2: 3.894
- S100A9: -0.189
- TDRD9: 1.923
- ZAP70: 2.482

8. The method of any one of claims 1 to 7, wherein the assayed biological sample(s) is (are) blood sample(s), preferably total blood sample(s).

9. The method of any one of claims 1 to 8 which is carried out on a population of assayed samples previously drawn from patients for which it is known for a part of the samples whether they have been drawn from patients that have presented at least one episode of infection since their admission in a care unit, notably their admission in an ICU, which further comprises:
a. Determining an optimal threshold value, which is associated to the transcriptomic score calculated in step c.ii of claim 1, through calculation of the Youden Index of the said transcriptomic score, in particular wherein the optimal threshold value is the value for which the Youden Index is maximized,
b. Optionally, providing the said optimal threshold value or the said Youden Index or both as output value(s).

10. A method to classify a sample previously drawn from a patient, in particular previously drawn from a patient admitted in an Intensive Care Unit (ICU), in a group reflecting a risk of adverse clinical outcome in a clinical setting, said method comprising the steps of:
a. Carrying out a method of determining a transcriptomic score (TScore) according to any one of claims 1 to 9 on a sample previously drawn from a patient in order to calculate a transcriptomic score, and
b. Comparing the calculated transcriptomic score of step a. to a predetermined threshold value, in particular a predetermined threshold value that is an optimal threshold value determined through calculation of a Youden index on a transcriptomic score obtained on a reference or training population of assayed samples, for example comparing the calculated transcriptomic score of step a. to an optimal cut-off value which a been predetermined with a method as defined in claim 9, and
c. From the comparison of step a., assigning the sample drawn from the patient to a group of samples reflecting a risk of adverse clinical outcome in a clinical setting, and
d. Optionally, providing the classification result obtained in step c. as an output value.

11. A method to identify a patient at risk of adverse clinical outcome in a clinical setting, comprising carrying out a method of classification according to claim 10 on a sample previously drawn from a patient, and identifying the patient as having a risk of adverse clinical outcome in a clinical setting on the basis of the basis of the group to which the sample has been assigned to.

12. A method according to any one of claims 1 to 11, wherein the adverse clinical outcome is:
- the occurrence of complications, such as a care unit-acquired infection, in particular the occurrence of an intensive care unit acquired infection (ICU-AI), notably a nosocomial infection, and/or
- a prolonged stay in care unit (in particular an ICU), and/or
- death.

13. The method of any one of claims 1 to 12, which is for:
a. *in vitro* or *ex vivo* diagnosing or prognosticating whether a patient is at risk of adverse clinical outcome in a clinical setting, in particular whether the patient is at risk of acquiring an intensive care unit acquired infection, in particular a nosocomial infection, and/or
b. *in vitro* or *ex vivo* monitoring the evolution of the health status of a patient over time, in particular when the method is carried out at several distinct points of time on several distinct samples drawn from the patient, the monitoring of the evolution consisting in comparing the conclusion reached at the different sampling times, and/or
c. *in vitro* or *ex vivo* classifying the patient as pertaining to a group displaying immune alterations in circulating cells, and/or *in vitro* or *ex vivo* diagnosing or prognosticating whether a patient has an immune dysfunction background.

14. The method of any one of claims 1 to 13, wherein when the following genes: C3AR1, CD177, CX3CR1, IFNgamma, IL1R2, S100A9, TDRD9 and ZAP70 are assayed, a risk of adverse clinical outcome is present when the transcriptomic score calculated for the patient assayed sample is equal or greater than 3.

15. An *in vitro* or *ex vivo* method of screening whether a drug has the capacity to alleviate an adverse clinical outcome, in particular an adverse clinical outcome as defined in claim 12, in a patient, comprising the steps of:
- Determining whether a patient is at risk of adverse clinical outcome in a clinical setting, by carrying out, at a first point in time, a method according to any one of claims 1 to 11 on a sample previously drawn from the patient, and
- If the patient is determined to be at risk of adverse clinical outcome in a clinical setting, reiterating, at a second point in time placed after the first point in time and after treatment of the said patient with a drug deemed to have the capacity to alleviate the adverse clinical outcome, the carrying out of a method according to any one of claims 1 to 11 on a sample previously drawn from such a patient at the second point in time that is later than the first point in time, and
- Optionally, concluding about the capacity of the drug to alleviate an adverse clinical outcome.

16. The method according to any one of claims 1 to 16, which is carried out by a computer.

17. A data processing apparatus comprising:
a. means for carrying out a method according to claim 16, especially means for retrieving gene expression values and/or means for inputting reference values, and/or means for determining ROC curves and/or means for determining if decision conditions are met or calculating decision conditions and/or means for calculating a transcriptomic score, and/or means to provide output values, optionally means for measuring gene expression values on a biological sample previously drawn from a patient, when said means are carried out or driven by a computer, or
b. comprising a processor adapted to/configured to perform a method according to claim 16, especially a processor adapted to/configured to perform the steps of a method referred to by claim 16.

18. A computer program comprising instructions which, when the program is executed by a computer or processor, cause the computer to carry out a method according to claim 16.

19. Use of a kit comprising means for amplifying and/or detecting gene expression values for at least two distinct genes selected among the genes defined in claim 5, in particular genes from the list: C3AR1, CD177, CD3D, CD74, CIITA, CTLA4, CX3CR1, IFNgamma, IL1R2, TAP2, S100A9 TDRD9 and ZAP70, for carrying out a method according to any one of claims 1 to 16, especially by measuring the gene expression values of at least two distinct genes selected among the genes defined in claim 5 in a biological sample previously removed from a human patient, in particular a human patient in a clinical settings, which has been admitted in an hospital, especially a human patient susceptible of being diagnosed as being at risk of sepsis or a patient susceptible of suffering of condition(s) than can evolve in a sepsis event.
